# FASCICULE DE BREVET EUROPEEN

(11) **EP 0 980 427 B1**
(45) Date de publication et mention de la délivrance du brevet: **25.01.2006**
(21) Numéro de dépôt: 98912541.4
(22) Date de dépôt: 19.02.1998
(51) Int. Cl.: C12N 15/12, C07K 14/47, C07K 16/18, G01N 33/574, C12Q 1/68, A61K 38/17, A61K 48/00, A61K 39/395

(54) **UTILISATION DES PROTEINES ULIP DANS LE DIAGNOSTIC ET LA THERAPIE DES CANCERS ET DES SYNDROMES NEUROLOGIQUES PARANEOPLASIQUES**
VERWENDUNG VON ULIP PROTEINEN ZUR DIAGNOSE UND THERAPIE VON KREBS UND PARANEOPLASTISCHEN NEUROLOGISCHEN SYNDROMEN
USE OF ULIP PROTEINS IN THE DIAGNOSIS AND THERAPY OF CANCER AND PARANEOPLASTIC NEUROLOGICAL SYNDROMES

(30) Priorité: 19.02.1997 FR 9701961
(43) Date de publication de la demande: 23.02.2000
(73) Titulaire: INSTITUT NATIONAL DE LA SANTE ET DE LA RECHERCHE MEDICALE (INSERM), 75013 Paris (FR)
(72) Inventeur: AGUERA, Michèle, F-69300 Caluire (FR); BELIN, Marie-Françoise, F-69005 Lyon (FR); HONNORAT, Jérôme, F-69500 Bron (FR); KOLATTUKUDY, Pappachan, Columbus, OH 43220 (US); QUACH, Than, Tam, F-69008 Lyon (FR); BYK, Tamara, F-94000 Créteil (FR); SOBEL, André, F-75013 Paris (FR); AUNIS, Dominique, F-67100 Strasbourg (FR); ANTOINE, Jean-Christophe, F-42600 Chalin Le Comtal (FR)
(74) Mandataire: Bernasconi, Jean Raymond
(86) Numéro de dépôt international: PCT/FR1998/000328
(87) Numéro de publication internationale: WO 1998/037192

(56) Documents cités:
- J¹RÔME HONNORAT ET AL.: "Antibodies to a subpopulation of glial cells and a 66kDa developmental protein in patients with paraneoplastic neurological syndromes" JOURNAL OF NEUROLOGY, NEUROSURGERY AND PSYCHIATRY, vol. 61, 1996, pages 270-278, XP002045119 cité dans la demande
- TAMARA BYK ET AL.: "Identification and molecular characterization of Unc-33-like phosphoprotein (Ulip), a putative mammalian homolog of the axonal guidance-associated unc-33 gene product" JOURNAL OF NEUROSCIENCE, vol. 16, no. 2, 15 janvier 1996, pages 688-701, XP002045120 cité dans la demande
- YOSHIO GOSHIMA ET AL.: "Collapsin-induced growth cone collapse mediated by an intracellular protein related to UNC-33" NATURE, vol. 376, no. 6540, 10 août 1995, LONDON GB, pages 509-514, XP002069770 cité dans la demande
- Base de données EMBL Numéro d'accès Q62950; 1 Novembre 1996 WANG, L. ET AL. XP002045123
- NAOKI HAMAJIMA ET AL.: "A novel gene family defined by human dihydropyriminidase and three related proteins with differential tissue distribution" GENE, vol. 180, no. 1/2, 1996, AMSTERDAM NL, pages 157-163, XP002045121
- Base de données EMBL entrée HS901245 Numéro d'accès H85901; 22 Novembre 1995 HILLIER L. ET AL.: "The WashU-Merck EST Project." XP002045124
- BYK T ET AL: "The Ulips: A growing protein family related to the axonal guidance associated UNC-33 protein." 26TH ANNUAL MEETING OF THE SOCIETY FOR NEUROSCIENCE, WASHINGTON, D.C., USA, NOVEMBER 16-21, 1996. SOCIETY FOR NEUROSCIENCE ABSTRACTS 22 (1-3). 1996. 1713. ISSN: 0190-5295, XP002045122
- JANE E. MINTURN ET AL.: "TOAD-64, a gene expressed early in neuronal differentiation in the rat, is related to unc-33, a C. elegans gene involved in axon outgrowth" THE JOURNAL OF NEUROSCIENCE, vol. 15, no. 10, octobre 1995, pages 6757-6766, XP002069771
- Base de données Trrod Numéro d'accès O08553; 1 Juillet 1997 BYK T. ET AL.:"ULIP2 Protein" XP002069773
- Base de donées Trrod Numéro d'accès O08554; 1 Juillet 1997 BYK T. ET AL.:"ULIP3 Protein" XP002069774
- Base de données Trrod Numéro d'accès O08886; 1 Juillet 1997 BYK T. ET AL.:"ULIP4 Protein" XP002069775
- GAETANO, CARLO ET AL: "Identification and characterization of a retinoic acid-regulated human homolog of the unc - 33 - like phosphoprotein gene (hUlip) from neuroblastoma cells" J. BIOL. CHEM. (1997), 272(18), 12195-12201 CODEN: JBCHA3;ISSN: 0021-9258, 1997, XP002069772

## Description

L'invention concerne l'utilisation des protéines dénommées ULIP/POP dans le diagnostic et la thérapie des cancers et des syndromes neurologiques paranéoplasiques.

Les syndromes neurologiques paranéoplasiques (SNP) surviennent à l'occasion d'un cancer, souvent avant sa découverte et ne sont liés ni à la prolifération tumorale elle-même (envahissement direct métastases) ni à la thérapie. Leur fréquence est globalement estimée à environ 1 % des cancers. Plusieurs tableaux cliniques ont été depuis longtemps individualisés (encéphalomyélite, neuropathie sensitive de Denny Brown, atrophie cérébelleuse, encéphalite limbique opsoclonus. ...) correspondant en fait à l'atteinte soit élective soit préférentielle de certains groupes de neurones. La fréquence des cellules inflammatoires au voisinage des lésions avait fait évoquer depuis de nombreuses années la possibilité d'un processus auto-immun ou viral. La mise en évidence, plus récente, d'auto-anticorps dans le sérum et le liquide céphalo-rachidien (LCR) de patients souffrant de SNP, spécifiques du type de tumeur et du type de neurones qui dégénèrent, a relancé l'hypothèse d'une participation de l'auto-immunité dans la génèse de cette pathologie (Graus et al., 1985 ; Greenlee et al., 1983).

Outre la présence d'un titre élevé de ces anticorps dans le sang et le LCR des patients, il existe plusieurs arguments suggérant que les SNP relèvent de mécanismes auto-immuns. Ainsi, les antigènes reconnus dans le système nerveux central sont aussi présents dans les tumeurs des patients (Anderson et al., 1987). Au sein du tissu tumoral, on retrouve des anticorps spécifiquement dirigés contre ces antigènes ainsi que des lymphocytes B et T (Hetzel et al., 1990).

Ces données suggèrent que le processus auto-immun serait déclenché par l'expression d'antigènes tumoraux. Un processus d'immunité croisée provoquerait les lésions du système nerveux central. D'autres arguments indiquent en outre que les lésions cérébrales résultent de la réponse auto-immune. Ainsi, dans le cerveau des patients, le titre des anticorps spécifiques est supérieur à celui du sérum et du LCR (Dalmau et al., 1991). De plus, dans le cas des encéphalomyélites associées aux anticorps anti-Hu, il existe une réaction lymphocytaire intense, composée de cellules B et T, située à proximité de neurones en voie de destruction (Dalmau et al., 1991 ; Graus et al., 1990).

Plusieurs types d'auto-anticorps permettant des regroupements syndromiques précis en fonction de critères immunologiques, neurologiques et carcinologiques ont été décrits.

Ainsi, les anticorps anti-Yo sont retrouvés dans le sérum et le LCR de femmes présentant une atrophie cérébelleuse paranéoplasique et un cancer gynécologique (ovaire, sein ou utérus) (Greenlee et al., 1983 ; Jaeckle et al., 1985).

Ces anticorps reconnaissent deux protéines cytoplasmiques de 34 et 62 kDa spécifiques des cellules de Purkinje du cervelet.

Les anticorps anti-Ri sont retrouvés dans le sérum et le LCR de patients (principalement des femmes) présentant un opso-myoclonus, un syndrome cérébelleux et un cancer du sein. Ces anticorps reconnaissent deux protéines de 50 et 80 kDa spécifiques des neurones du système nerveux central (Luque et al., 1991).

Les anticorps anti-Hu sont les plus fréquemment rencontrés au cours des SNP. Ils sont retrouvés dans le sérum et le LCR de patients présentant un syndrome de Denny-Brown ou une encéphalomyélonévrite et un cancer du poumon à petites cellules (Graus et al., 1985 ; Dalmau et al., 1992). Ces auto-anticorps reconnaissent plusieurs protéines de 37 à 45 kDa exprimées spécifiquement par l'ensemble des neurones du système nerveux.

Récemment a été identifié chez des patients présentant un SNP un autre type d'auto-anticorps : les anticorps anti-CV2 (Antoine et al., 1993 ; Honnorat et al., 1996). Ces derniers sont atypiques, en ce sens que la cible antigénique reconnue à l'âge adulte est essentiellement non neuronale, alors que l'analyse du cerveau *post-mortem* de quatre patients permet d'objectiver une perte neuronale, une gliose et un processus inflammatoire caractéristique des SNP.

L'originalité de la découverte de ces auto-anticorps réside, d'une part, dans leur mise en évidence. Ces derniers avaient échappé à l'ensemble des investigations habituelles qui consistaient à révéler les antigènes reconnus par immunohistochimie sur du cerveau *post-mortem.* L'antigène reconnu est en effet soluble et disparaît du cerveau *post-mortem* dans la plupart des conditions de fixation. Seule une fixation du tissu *post-mortem* humain par immersion dans le paraformaldéhyde ou *in situ* par perfusion de paraformaldéhyde chez l'animal, a permis de révéler la présence de ces anticorps dans le LCR ou le sérum des patients atteints de SNP (Antoine et al., 1993 ; Honnorat et al., 1996).

Les auto-anticorps anti-CV2 présents dans les sérums de patients atteints de syndrome neurologique paranéoplasique (SNP) ont été définis par leur capacité à reconnaître, par immunohistochimie indirecte, un antigène cytoplasmique exprimé spécifiquement, dans le cerveau de rat adulte, par une sous-population d'oligodendrocytes du tronc cérébral, de la moelle et du cervelet.

L'originalité de ces auto-anticorps réside, d'autre part, dans leur intérêt diagnostique. Leur présence dans le sérum ou le LCR de patients a valeur diagnostique puisqu'elle permet de préciser l'origine paranéoplasique d'un syndrome neurologique. La découverte de ces anticorps lorsqu'elle précède celle du cancer, oriente la recherche de celui-ci et permet sa découverte. Tel a été le cas pour six patients sur 19 présentant des anticorps anti-CV2. Les troubles cliniques étaient différents suivant les patients, certains présentaient un tableau d'encéphalite limbique, d'autres une encéphalomyélonévrite et d'autres un syndrome de Lambert-Eaton. Néanmoins, dans plus de 60 % des cas, le syndrome cérébelleux était prédominant. La tumeur la plus fréquemment associée était le cancer du poumon à petites cellules (60 % des cas).

Des expériences sur des cerveaux de rats nouveaux-nés ont montré que ces anticorps anti-CV2 réagissaient avec une protéine de 66 kDa (Honnorat et al., 1996).

Cet antigène se situe dans le cerveau adulte dans une sous-population d'oligodendrocytes ou dans des cellules qui gardent des capacités de différenciation dans le cerveau adulte (bulbe olfactif, gyrus denté). L'antigène reconnu jouerait un rôle dans la survie neuronale, via des interactions Neurone/Oligodendrocyte, comme le suggère la perte des neurones observée dans le cerveau *post-mortem* de patients atteints de SNP.

Son expression très restreinte à l'âge adulte contraste avec une expression très forte et transitoire dans le système nerveux central et périphérique en développement, suggérant le rôle probable de cet antigène dans le développement du système nerveux.

La Demanderesse a caractérisé l'antigène cible des auto-anticorps anti-CV2 qui correspond à une protéine ci-après désignée par «POP-66» pour « paraneoplastic oligodendrocyte protein 66 kDa ».

De manière surprenante, il a été découvert que la protéine POP-66 appartient à la famille des protéines dites ULIP (pour Unc-33 like phosphoprotein), impliquée dans le contrôle du développement neuronal et le transport axonal, (T. Byk et al., 1996) et étudiée aussi sous la forme des protéines CRMP (Goshima et al., 1995, Wang et al., 1996), TOAD-64 (Minturn et al., 1995) et DRPs (Hamajima et al., 1996). Plus précisément, POP-66 a été identifiée comme étant en fait la forme humaine de ULIP-4.

L'ensemble des données décrit ci-après souligne la complexité de cette famille de protéines, l'existence d'un spectre d'expression très large des membres de cette famille dans le cerveau au cours de l'ontogénèse, mais très restreint chez l'adulte, ainsi que la spécificité des anticorps anti-CV2 pour un membre de cette famille protéique ULIP, qui est en fait POP-66.

Ainsi, la Demanderesse a montré que la protéine reconnue par les anticorps anti-CV2 de patients atteints de SNP est POP-66/ULIP-4 et a établi l'implication des protéines ULIP dans les syndromes neurologiques paranéoplasiques et les cancers associés. Outre leur rôle dans les cancers associés aux SNP, la Demanderesse a également découvert que les protéines de la famille ULIP pourraient jouer un rôle dans toute autre forme de cancer, non associée aux SNP. Plus particulièrement, les protéines ULIP seraient notamment impliquées dans les cancers de tissus ayant une origine embryonnaire commune avec le système nerveux central.

La présente invention a donc pour objet un polypeptide purifié ULIP, dérivé ou fragment polypeptidique dudit polypeptide purifié comprenant une séquence d'acides aminés SEQ ID n° 8.

De manière préférentielle, la présente invention a pour objet un polypeptide purifié, dérivé, ou fragment polypeptidique biologiquement actif dudit polypeptide purifié, comprenant la séquence d'acides aminés SEQ ID n° 8, ledit polypeptide étant désigné par « POP-66/ULIP-4 ».

Un fragment du polypeptide de séquence SEQ ID n° 8 d'intérêt est en particulier le fragment antigénique PARASCPGKIS (acides aminés n° 517 à n° 527).

Dans la description de l'invention, on utilise les définitions suivantes :
- dérivé : tout polypeptide variant du polypeptide de séquence SEQ ID n° 8 ou toute autre molécule résultant d'une modification de nature génétique et/ou chimique de la séquence SEQ ID n° 8, c'est-à-dire obtenue par mutation, délétion, addition, substitution et/ou modification chimique d'un seul ou d'un nombre limité d'acides aminés, ainsi que toute séquence isoforme, c'est-à-dire une séquence identique à la séquence SEQ ID n° 8 à l'un de ses fragments ou séquences modifiées, contenant un ou plusieurs acides aminés sous la forme d'énantiomère D. lesdites séquences variantes modifiées ou isoformes ayant conservé au moins l'une des propriétés les rendant biologiquement actives.
- Biologiquement actif : présentant des propriétés d'induction et/ou de contrôle du développement neuronal et/ou des propriétés antigéniques.

L'invention a également pour objet une séquence d'acides nucléiques isolée SEQ ID n°7 ou une séquence dérivée de la séquence SEQ ID n°7 du fait de la dégénérescence du code génétique, ou du fait de mutation, de délétion ou d'insertion d'au moins un nucléotide, lesdites séquences dérivées ayant une activité biologique pratiquement identique à celle du peptide codé par la séquence SEQ ID n°7.

Les différentes séquences nucléotidiques de l'invention peuvent être d'origine artificielle ou non. Il peut s'agir de séquences d'ADN ou d'ARN, obtenues par criblage de banques de séquences au moyen de sondes élaborées sur la base de la séquence SEQ ID n° 8. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage des banques.

Ces séquences nucléotidiques permettent la réalisation de sondes nucléotidiques, capables de s'hybrider fortement et spécifiquement avec une séquence d'acides nucléiques, d'un ADN génomique ou d'un ARN messager, codant pour un peptide selon l'invention ou un fragment biologiquement actif de celui-ci. Les conditions d'hybridation appropriées correspondent aux conditions de température et de force ionique usuellement utilisées par l'homme du métier (Sambrook et al., 1989), de préférence à des conditions de température comprises entre (Tₘ moins 5°C) et (Tₘ moins 30°C) et de préférence encore, à des conditions de température comprises entre (Tₘ moins 5°C) et (Tₘ moins 10°C) (forte stringence), Tm étant la température théorique de fusion, définie comme étant la température à laquelle 50 % des brins appariés se séparent. De telles sondes font également partie de l'invention. Elles peuvent être utilisées comme outil de diagnostic *in vitro* pour la détection, par des expériences d'hybridation, de transcrits spécifiques des polypeptides de l'invention dans des échantillons biologiques ou pour la mise en évidence de synthèses aberrantes ou d'anomalies génétiques résultant d'un polymorphisme, de mutations ou d'un mauvais épissage.

Les sondes de l'invention comportent au minimum 10 nucléoüdes, et au maximum comportent la totalité d'une séquence nucléotidique SEQ ID n° 7 ou de son brin complémentaire.

Les méthodes de diagnostic *in vitro* dans lesquelles ces sondes nucléotidiques sont mises en oeuvre pour la détection de synthèses aberrantes ou d'anomalies génétiques, telles que la perte d'hétérozygotie et le réarrangement génétique, au niveau des séquences nucléiques codant pour un polypeptide ULIP selon l'invention ou un fragment biologiquement actif, sont incluses dans la présente invention. Un tel type de méthode comprend :
- la mise en contact d'une sonde nucléotidique de l'invention avec un échantillon biologique dans des conditions permettant la formation d'un complexe d'hybridation entre ladite sonde et la susdite séquence nucléotidique, éventuellement après une étape préalable d'amplification de la susdite séquence nucléotidique ;
- la détection du complexe d'hybridation éventuellement formé :
- éventuellement le séquençage de la séquence nucléotidique formant le complexe d'hybridation avec la sonde de l'invention.

Les sondes d'ADNc de l'invention sont en outre avantageusement utilisables pour la détection d'anomalies chromosomiques.

Les séquences nucléotidiques selon l'invention sont également utiles pour la réalisation et l'utilisation d'amorces oligonucléotidiques sens et/ou antisens pour des réactions de séquençage ou d'amplification spécifique selon la technique dite de PCR (réaction de polymérisation en chaîne) ou toute autre variante de celle-ci.

Les séquences nucléotidiques selon l'invention ont par ailleurs des utilisations dans le domaine thérapeutique, pour la réalisation de séquences antisens, capables de s'hybrider spécifiquement avec une séquence d'acide nucléique, y compris un ARN messager, utilisables en thérapie génique. L'invention a ainsi pour objet des séquences antisens capables d'inhiber, au moins partiellement, la production d'un polypeptide selon l'invention, tel que défini précédemment.

Elles sont plus particulièrement utiles dans le traitement des désordres du système nerveux central et périphérique et de la vision, notamment dans le traitement des syndromes neurologiques paranéoplasiques, ainsi que dans le traitement anticancéreux, notamment des tumeurs associées à des syndromes neurologiques paranéoplasiques.

Les séquences nucléotidiques selon l'invention peuvent par ailleurs être utilisées pour la production de protéines recombinantes ULIP selon l'invention.

Ces protéines peuvent être produites à partir des séquences nucléotidiques définies ci-dessus, selon des techniques de production de produits recombinants connues de l'homme du métier. Dans ce cas, la séquence nucléotidique utilisée est placée sous le contrôle de signaux permettant son expression dans un hôte cellulaire.

Un système efficace de production d'une protéine recombinante nécessite de disposer d'un vecteur, par exemple d'origine plasmidique ou virale, et d'une cellule hôte compatible.

L'hôte cellulaire peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible. Un hôte cellulaire préféré pour l'expression des protéines de l'invention est constitué par la bactérie *E*. *coli.*

Le vecteur doit comporter un promoteur, des signaux d'initiation et de terminaison de la traduction, ainsi que les régions appropriées de régulation de la transcription. Il doit pouvoir être maintenu de façon stable dans la cellule et peut éventuellement posséder des signaux particuliers spécifiant la sécrétion de la protéine traduite.

Ces différents signaux de contrôle sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation.

L'invention vise en outre les cellules hôtes transfectées par ces vecteurs précédents. Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Ces cellules sont utilisables dans une méthode de production d'un polypeptide recombinant selon l'invention ou tout fragment ou dérivé biologiquement actif de celui-ci.

La méthode de production d'un polypeptide de l'invention sous forme recombinante est elle-même comprise dans la présente invention, et se caractérise en ce que l'on cultive les cellules transfectées dans des conditions permettant l'expression d'un polypeptide recombinant selon l'invention ou de tout fragment ou dérivé biologiquement actif de celui-ci, et que l'on récupère ledit polypeptide recombinant.

Les procédés de purification utilisés sont connus de l'homme du métier. Le polypeptide recombinant peut être purifié à partir de lysats et extraits cellulaires, du surnageant du milieu de culture, par des méthodes utilisées séparément ou en combinaison, telles que le fractionnement, les méthodes de chromatographie, les techniques d'immunoaffinité à l'aide d'anticorps mono ou polyclonaux spécifiques, etc.

Une variante consiste à produire un polypeptide recombinant fusionné à une protéine "porteuse" (protéine chimère). L'avantage de ce système est qu'il permet une stabilisation et une diminution de la protéolyse du produit recombinant, une augmentation de la solubilité au cours de la renaturation *in vitro* et/ou une simplification de la purification lorsque le partenaire de fusion possède une affinité pour un ligand spécifique.

L'exploitation des protéines ULIP, et en particulier POP-66/ULIP-4, ainsi que des anticorps dirigés contre ces protéines, est prometteuse dans divers domaines.

Ainsi, la détection de l'auto-anticorps anti-CV2 par immunofluorescence sur cerveau animal fixé est utilisée actuellement comme test diagnostic.

La production de protéine recombinante POP-66/ULIP-4 selon l'invention permet la fabrication d'un test (de type Elisa ou Western Blot) rapide et fiable, pour détecter les anticorps anti-CV2.

De tels tests existent déjà pour les anticorps anti-Hu, anti-Yo et anti-Ri. Le test pour détecter les anti-CV2 dans le sérum des patients pourrait être prescrit en cas de suspicion de syndrome neurologique paranéoplasique et inclurait par conséquent les anticorps anti-CV2 au même titre que les autres anticorps identifiés dans les SNP tels que précédemment cités.

L'invention vise donc également une méthode pour le diagnostic des syndromes neurologiques paranéoplasiques et/ou pour le diagnostic précoce de la formation de tumeurs d'origine cancéreuse, caractérisée en ce que l'on met en évidence dans un échantillon de sang prélevé chez un individu des auto-anticorps dirigés contre une protéine POP-66/ULIP-4 par
- la mise en contact un échantillon de sang prélevé chez un individu avec un polypeptide purifié (POP-66), dérivé ou fragment polypeptidique biologiquement actif de POP-66/ULIP-4 éventuellement fixé sur un support dans des conditions permettant la formation de complexes immunologiques spécifiques entre ledit polypeptide et les auto-anticorps éventuellement présent dans l'échantillon de sérum, et
- la détection des complexes immunologiques spécifiques éventuellement formés.

L'invention a également pour objet un kit pour le diagnostic des syndromes neurologiques paranéoplasiques et pour le diagnostic précoce de la formation des tumeurs à partir d'un prélèvement biologique comprenant :
- au moins un polypeptide purifié POP-66/ULIP-4. dérivé ou fragment polypeptidique biologiquement actif de POP-66/ULIP-4, éventuellement fixé sur un support,
- des moyens de révélation de la formation de complexes antigène/anticorps spécifiques entre un auto-anticorps anti-POP-66 et ledit polypeptide purifié POP-66, dérivé ou fragment polypeptidique et/ou des moyens de quantification de ces complexes.

L'invention a également pour objet les anticorps mono-clonaux ou leurs fragments, Fab ou F(ab')_{2,} ou immunoconjugués, dirigés contre un polypeptide purifié ULIP comprenant une séquence d'acides aminés SEQ ID n° 8, dérivé ou fragment polypeptidique biologiquement actif de ULIP et leur utilisation, pour la purification ou la détection d'une protéine ULIP dans un échantillon biologique.

Des anticorps polyclonaux peuvent être obtenus à partir du sérum d'un animal immunisé contre la protéine, produite par exemple par recombinaison génétique suivant la méthode décrite ci-dessus, selon les modes opératoires usuels.

Les anticorps monoclonaux peuvent être obtenus selon la méthode classique de culture d'hybridomes décrite par Kôhler et Milstein.

Les anticorps peuvent être des anticorps humanisés, des fragments Fab et F(ab')2. Ils peuvent également se présenter sous forme d'immunoconjugués ou d'anticorps marqués.

Ainsi, les anticorps dirigés contre une protéine de la famille ULIP sont utiles pour détecter une expression anormale de protéine ULIP chez des patients présentant des syndromes neurologiques, chez qui aucun cancer n'a été diagnostiqué par les méthodes classiques. Cette expression anormale de protéine ULIP pourra être corrélée à l'existence d'un cancer qui n'avait pas été repéré. Ainsi, les anticorps dirigés contre une protéine ULIP, notamment contre POP-66/ULIP-4, sont utiles pour le diagnostic précoce d'un cancer.

Une méthode de détermination d'une variabilité allélique, d'une mutation, d'une délétion, d'une insertion, d'une perte d'hétérozygotie ou d'une anomalie génétique du gène POP-66/ULIP-4, situé sur le chromosome 10 dans la région 26q pouvant être impliquées dans des pathologies, met en oeuvre au moins une séquence nuclèotidique SEQ ID n° 7. Parmi les méthodes de détermination d'une variabilité allélique, d'une mutation, d'une délétion, d'une insertion, d'une perte d'hétérozygotie ou d'une anomalie génétique du gène POP-66/ULIP-4, on préfère une méthode comprenant au moins une étape d'amplification par PCR de la séquence nucléique de POP-66/ULIP-4 susceptible de présenter un polymorphisme, une mutation, une délétion ou une insertion, à l'aide de couple d'amorces de séquences nucléotidiques, une étape au cours de laquelle on procède au traitement des produits amplifiés à l'aide d'enzymes de restriction appropriées et une étape au cours de laquelle on procède à la détection ou au dosage d'au moins l'un des produits de la réaction enzymatique.

De manière avantageuse, on peut rechercher les mutations associées audit chromosome 10 en relation avec le cancer, notamment les tumeurs cancéreuses périphériques et les tumeurs cérébrales primitives d'origine gliale par exemple.

L'invention a également pour objet une composition pharmaceutique comprenant au moins une protéine purifiée POP-66, fragment polypeptidique ou dérivé biologiquement actif de celle-ci, une séquence ou fragment de séquence nucléotidique codant pour ladite protéine, une séquence anti-sens capable de s'hybrider spécifiquement avec une séquence nucléotidique codant pour ladite protéine, ou un anticorps dirigé contre ladite protéine, associée à un véhicule pharmaceutiquement acceptable.

L'invention comprend de manière préférentielle des compositions pharmaceutiques comprenant comme principe actif un polypeptide POP-66 purifié, dérivé ou fragment polypeptidique de POP-66, préférentiellement sous forme soluble, associé à un véhicule pharmaceutiquement acceptable.

De telles compositions offrent une nouvelle approche pour traiter les désordres du système nerveux central et périphérique et de la vision, et notamment les syndromes neurologiques paranéoplasiques. Par ailleurs, elles sont utiles pour traiter les désordres neurologiques liés à une perte neuronale et/ou une sous-expression des protéines ULIP dans le système nerveux.

Ainsi, POP-66/ULIP-4 révèle aussi un intérêt dans des pathologies neurodégénératives telles que les atrophies multisystémiques qui sont des affections similaires à celles des SNP et pour lesquelles une anomalie d'une sous-population oligodendrocytaire a été détectée (Papp et al., 1992).

Les compositions selon l'invention sont par ailleurs utiles en thérapie anticancéreuse.

Les anticorps dirigés contre une ou plusieurs protéines ULIP peuvent être associés à des agents antinéoplasiques, permettant ainsi le ciblage des médicaments vers les cellules tumorales.

Ils peuvent en outre être associés à un groupe chimique hydrophile choisi de manière à passer ou non la barrière hémato-encéphalique, selon le type de tumeur.

Les protéines ULIP et en particulier POP-66 ainsi que les séquences nucléotidiques codant pour lesdites protéines et les séquences ou oligonucléotides anti-sens, peuvent être utiles dans la thérapie de tout type de cancer dans lequel un gène codant pour une protéine ULIP est impliqué. Parmi des exemples de cancers, on peut citer les tumeurs périphériques, telles que le cancer du poumon à petites cellules, le thymome, le cancer du sein et de l'ovaire, ainsi que les tumeurs cérébrales, de préférence les tumeurs cérébrales primitives d'origine gliale. L'expression de POP-66 dans les cellules non prolifératives du cerveau normal, son absence dans des tissus normaux tels que poumon ou thymus par exemple, sa réexpression différentielle lors de la tumorigénèse de ces tissus et la modulation de son expression dans une lignée tumorale au cours de la différenciation suggèrent à cet égard que POP-66 pourrait être un gène suppresseur de tumeur.

Préférentiellement, les compositions pharmaceutiques selon l'invention peuvent être administrées par voie systémique, de préférence par voie intraveineuse, par voie intramusculaire, intradermique ou par voie orale.

Leurs modes d'administration, posologies et formes galéniques optimaux peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés, etc.

Une protéine purifiée de la famille ULIP, fragment polypeptidique ou dérivé biologiquement actif de celle-ci, une séquence ou fragment de séquence nucléotidique codant pour ladite protéine, une séquence anti-sens capable de s'hybrider spécifiquement avec une séquence nucléotidique codant pour ladite protéine, ou un anticorps dirigé contre ladite protéine associée à un véhicule pharmaceutiquement acceptable, peuvent être utilisées pour la fabrication d'un médicament destiné à traiter les maladies neurodégénératives et les néoplasmes.

Une méthode de traitement des maladies neurodégénératives et des néoplasmes met en oeuvre l'administration à un sujet nécessitant un tel traitement d'une quantité thérapeutiquement efficace d'une protéine purifiée de la famille ULIP, fragment polypeptidique ou dérivé biologiquement actif de celle-ci, une séquence ou fragment de séquence nucléotidique codant pour ladite protéine, une séquence anti-sens capable de s'hybrider spécifiquement avec une séquence nucléotidique codant pour ladite protéine, ou un anticorps dirigé contre ladite protéine, associée à un véhicule pharmaceutiquement acceptable.

Les exemples et les figures dont les légendes sont présentées ci-après sont donnés à titre illustratif.

### LEGENDE DES FIGURES

- La figure 1 représente un profil d'électrophorèse à deux dimensions obtenu à partir d'extraits protéiques de cerveaux de rats nouveaux-nés enrichis en POP-66.
   A : coloration à l'argent de l'ensemble des protéines.
   B : immunoempreinte avec le sérum anti-CV2 de patients.

   Les flèches indiquent les taches correspondant à POP-66, révélées avec les anticorps anti-CV2.
- La figure 2 représente un profil d'électrophorèse à deux dimensions obtenue à partir d'extraits protéiques de cerveaux de rats nouveaux-nés.
   Immunoempreinte avec A- l'anticorps antipeptide 3 et B-l'anticorps anti-CV2.
- La figure 3 représente une électrophorèse à une dimension obtenue à partir d'extraits protéiques de cerveaux de rats nouveaux-nés.
   Immunoempreinte avec a : sérum préimmun pour peptide 3
   Immunoempreinte avec b : sérum anti-peptide 3
   Immunoempreinte avec c : sérum anti-peptide 4
   Immunoempreinte avec d : sérum préimmun pour peptide 4.
- La figure 4 représente un marquage immunohistochimique de coupes de cerveaux de rats adultes avec
   A : sérum anti-CV2 de patient atteint de SNP
   B : sérum de lapin avec des anticorps anti-peptide 3
   C : sérum de lapin avec des anticorps anti-peptide 4.
- La figure 5 représente un marquage histologique de coupes de cervelet de raton à 8 jours post-natal.
   A : coloration au bleu de toluidine ; ge = couche granulaire externe : m = couche moléculaire (x400).
   B : Immunomarquage après incorporation de BrdU (bromodéoxyuridine). Les cellules ayant incorporé le BrdU sont pratiquement toutes situées dans la zone la plus externe de la couche granulaire externe (ge). Quelques cellules positives sont situées dans la couche granulaire interne (x400).
   C : Immunoperoxydase indirecte avec un sérum de patient contenant un anticorps anti-CV2 (x400). L'immunoréactivité est concentrée dans la partie interne de la couche granulaire externe (future couche moléculaire (m)). Quelques cellules sont immunoréactives dans la couche granulaire interne. Les cellules de Purkinje (p) sont négatives ainsi que les cellules de la partie externe de la couche granulaire externe (ge).
   D : Immunoperoxydase indirecte avec un sérum de patient contenant un anticorps anti-CV2 (x1000). L'immunomarquage est surtout concentré dans la partie interne de la couche granulaire externe (future couche moléculaire (m)). On note une cellule réactive dans la couche granulaire interne (gi) (flêche).

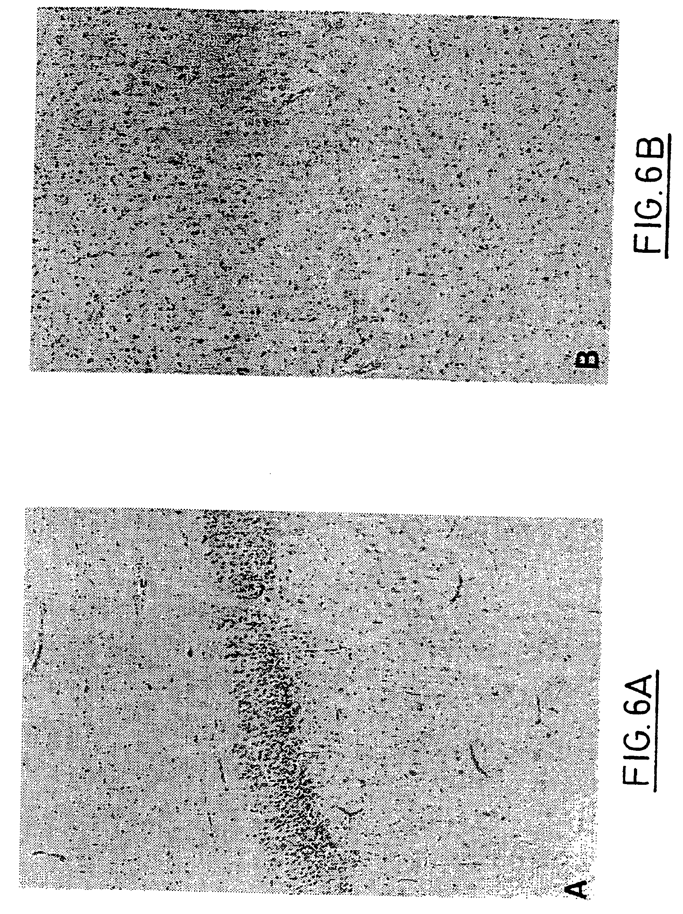

- La figure 6 représente un marquage immunohistochimique de coupes d'hippocampe humain post-mortem (coloration HPS).
   A : cerveau de patient témoin,
   B : cerveau de patient présentant une encéphalite limbique, et anticorps circulant anti-CV2. On peut noter la disparition des cellules granulaires.

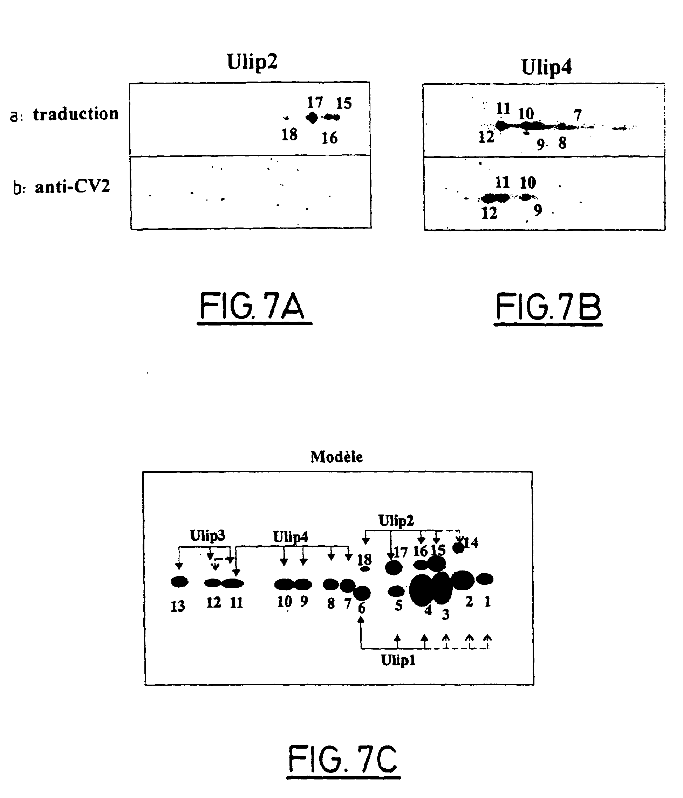

- La figure 7 représente un profil d'électrophorèse à deux dimensions avec la protéine ULIP-2 (A) contrôle et la protéine ULIP-4 (B).
   La figure 7C représente le modèle de profil de migration des protéines ULIP-1, 2, 3 et 4 comme référence.
   Les protéines sont révélées :
   a) par autoradiographie pour localiser les protéines traduites *in vitro* (traduction) ;
   b) par immunoempreinte avec le sérum anti-CV2.

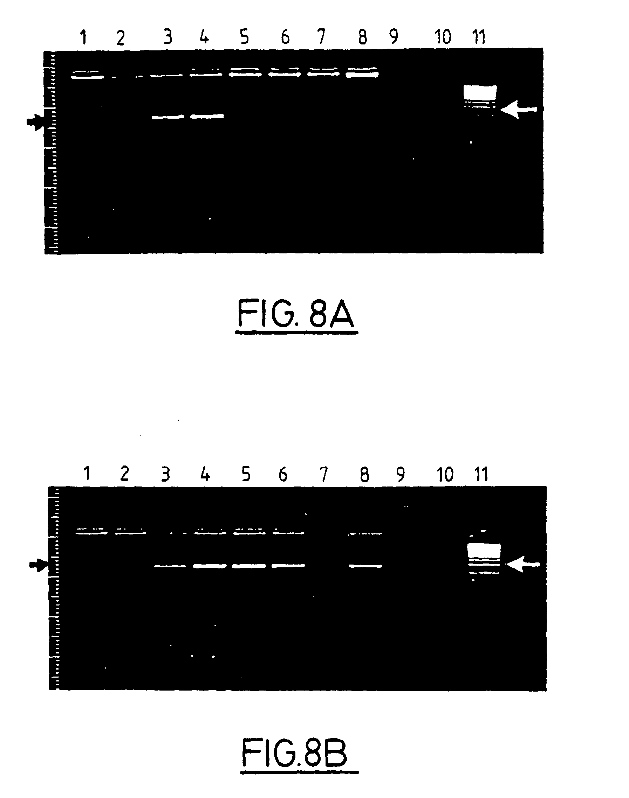

- La figure 8 représente un profil de migration de l'ARNm de C-22/ULIP-3 (8A) et TOAD-64/ULIP-2 (8B) amplifié par RT-PCR exprimé dans différents types cellulaires :
   pistes 1-3 : tumeur à petites cellules du poumon
   piste 2 : tumeur à petites cellules du poumon avec du sérum anti-CV2
   piste 4 : cDNA contrôle.
   piste 5 : médulloblastome traité par infection HTLV1
   pistes 6-7 : médulloblastome
   piste 8 : lignée C6 de cellules gliales chez la souris
   piste 9 : contrôle
   piste 10 : néant
   piste 11 : échelle kb.

   Les flèches noires correspondent à POP-66, les flèches blanches correspondent au standard de poids moléculaire.

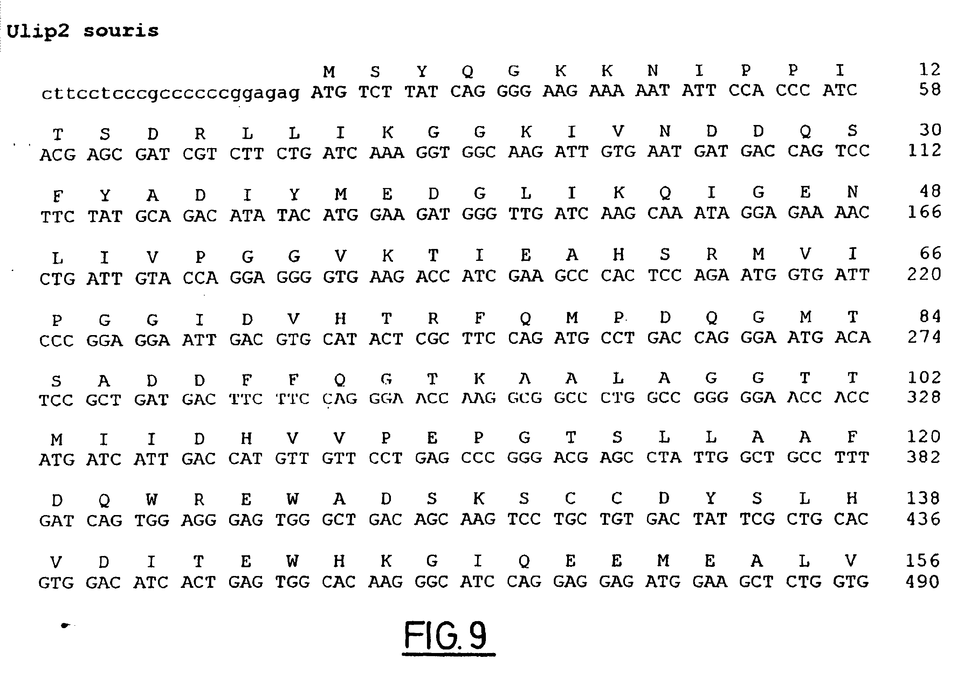

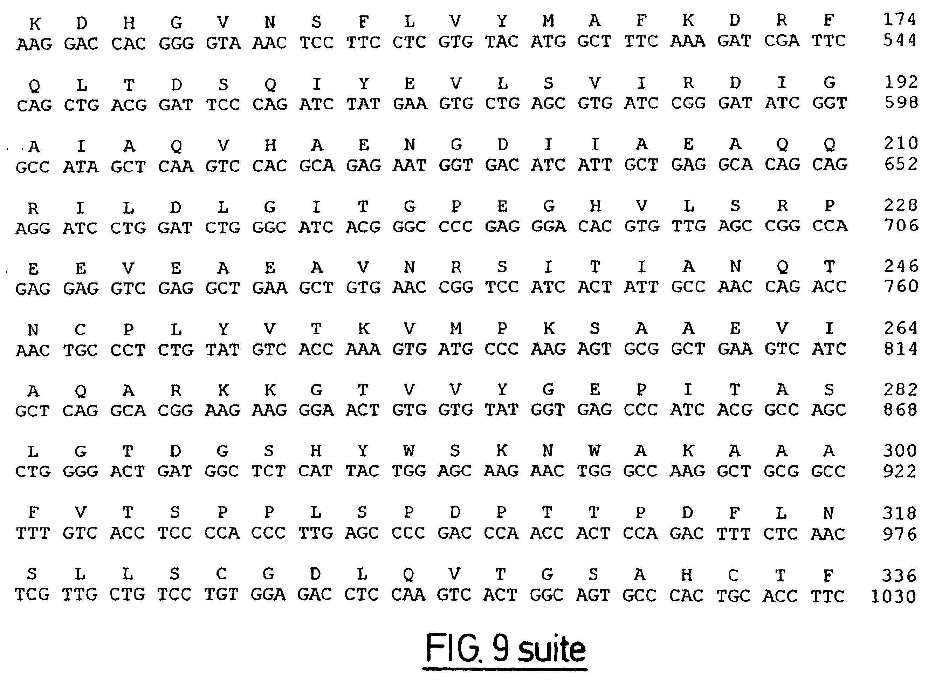

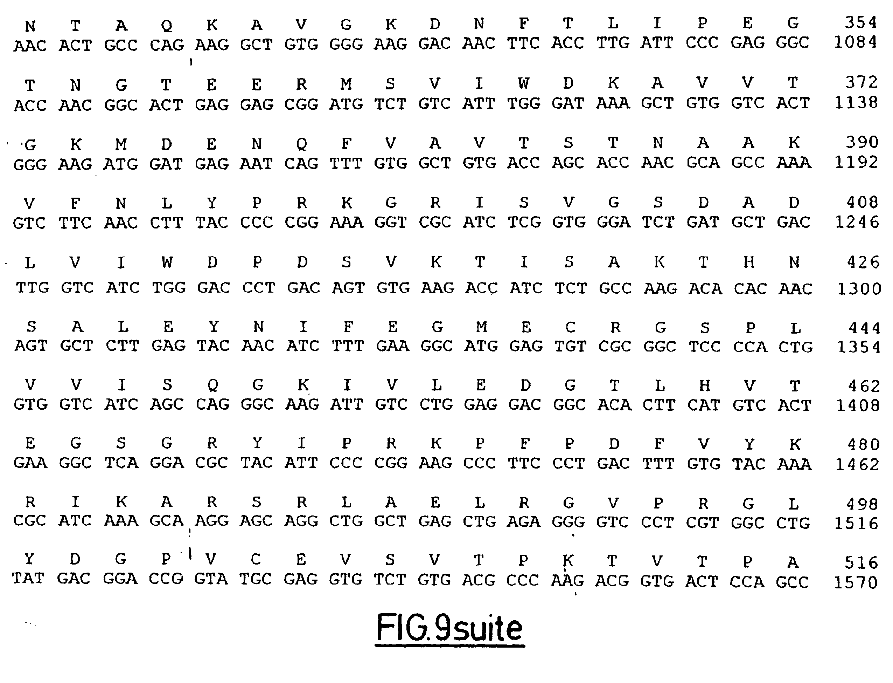

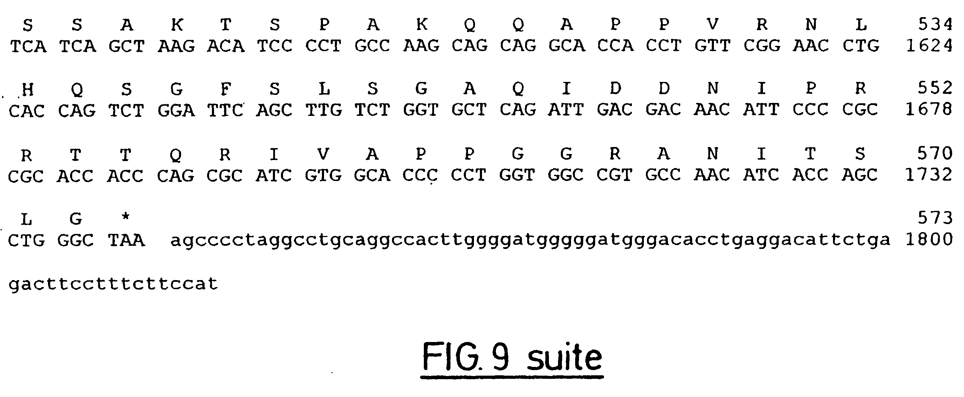

- La figure 9 représente la séquence nucléotidique d'ULlP-2 chez la souris (SEQ ID N° 1), ainsi que la séquence en acides aminés déduite (SEQ ID N° 2).

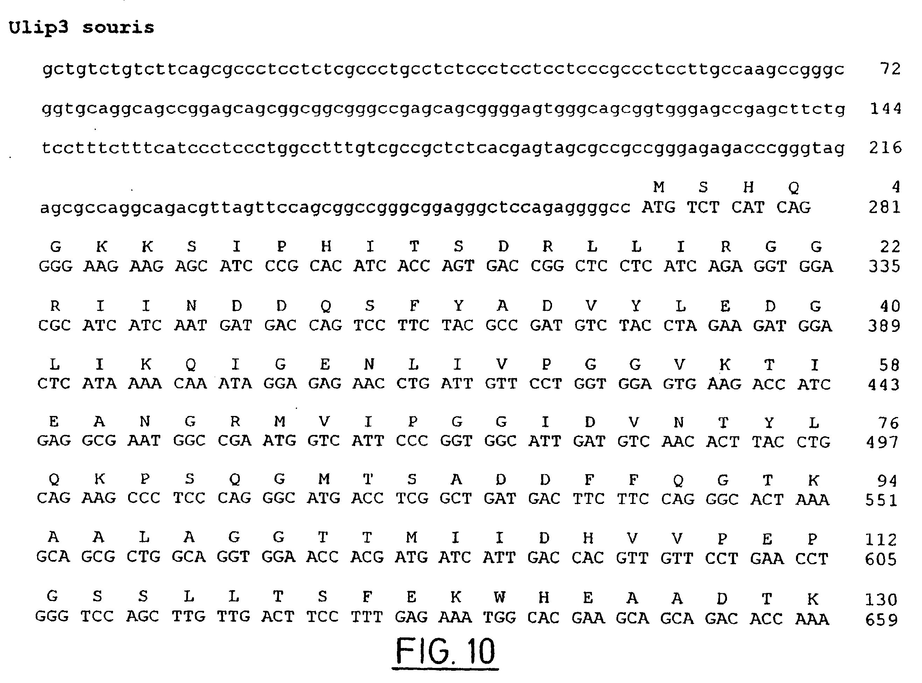

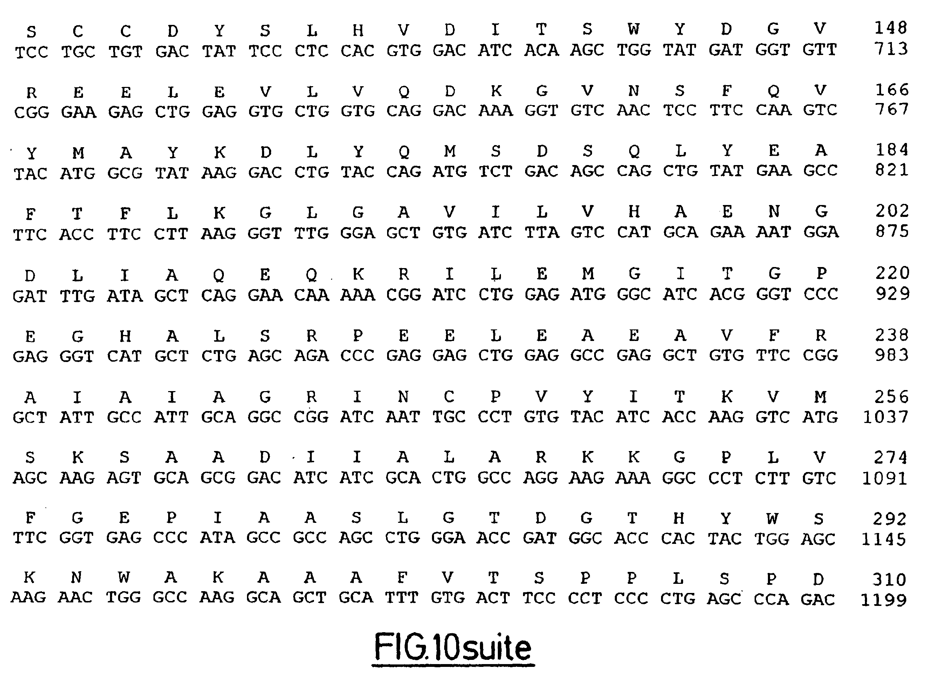

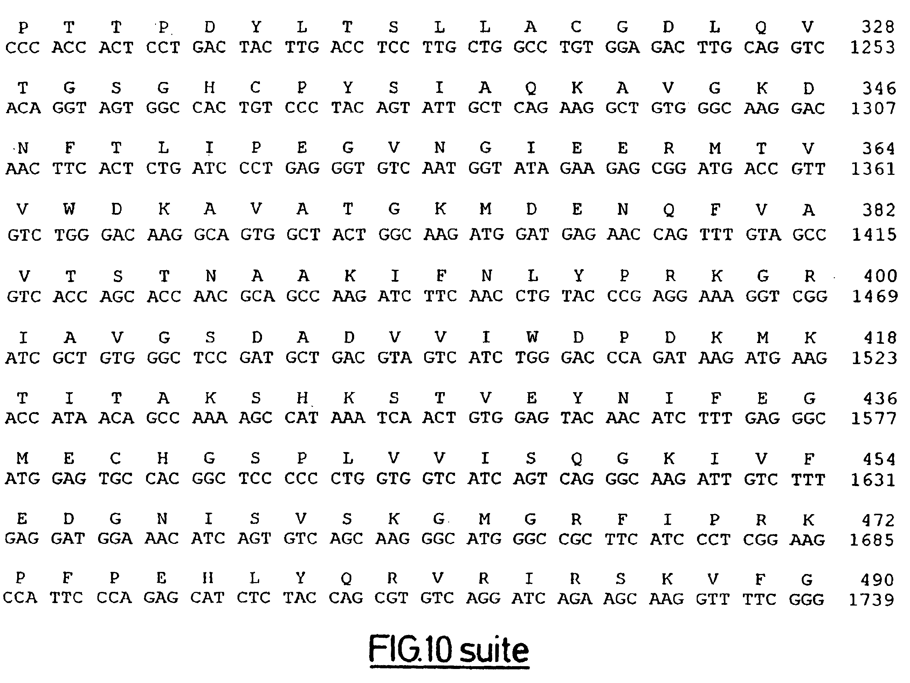

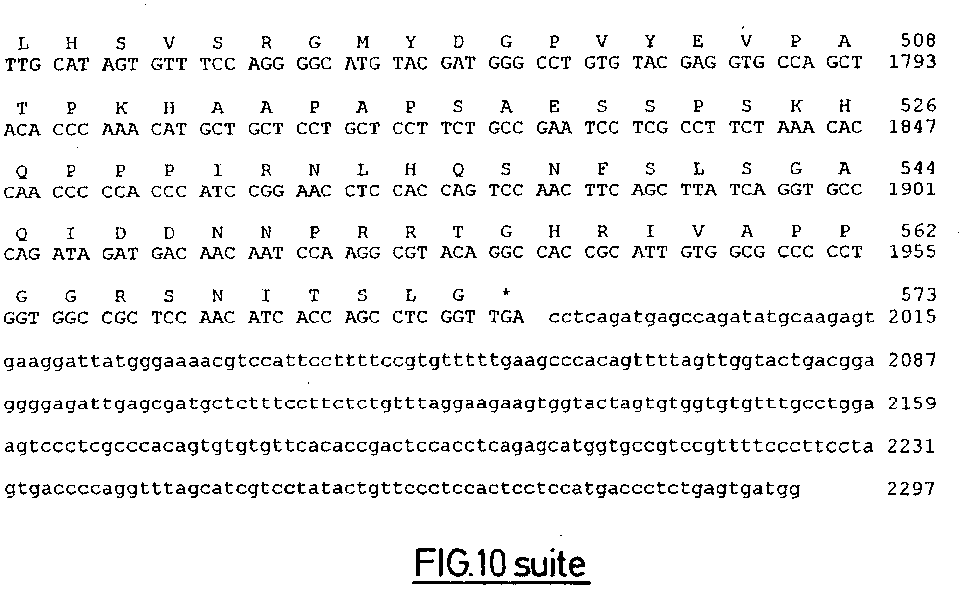

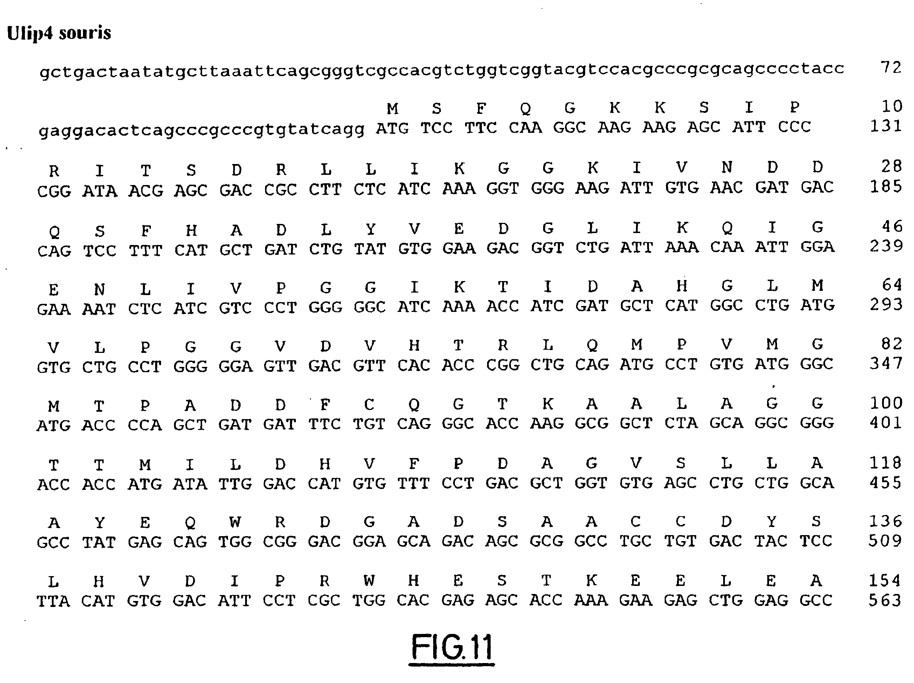

- La figure 10 représente la séquence nucléotidique d'ULIP-3 chez la souris (SEQ 10 N° 3), ainsi que la séquence en acides aminés déduite (SEQ ID N° 4).

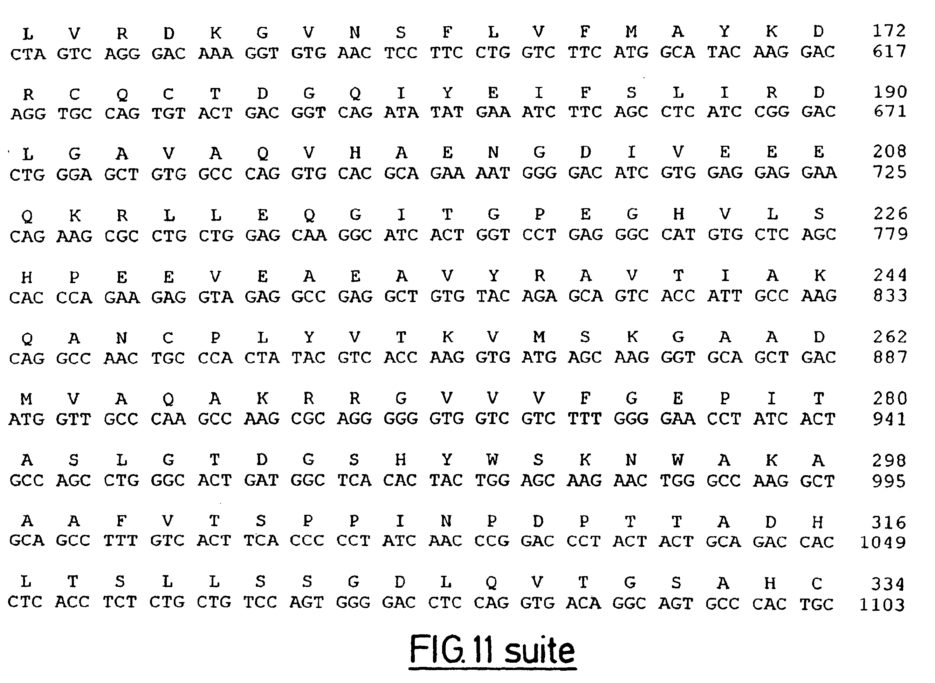

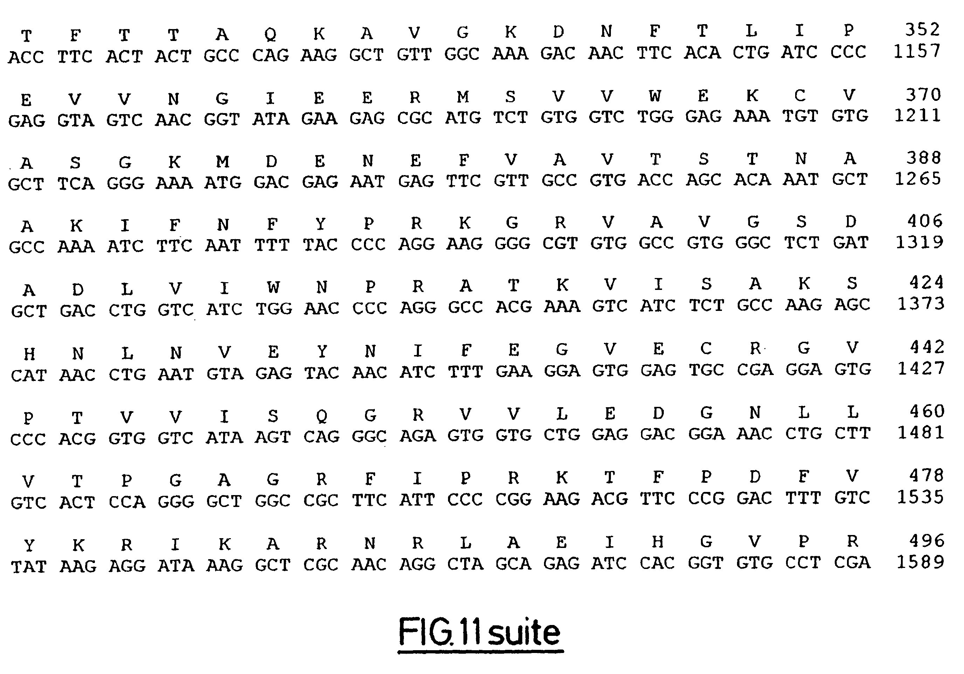

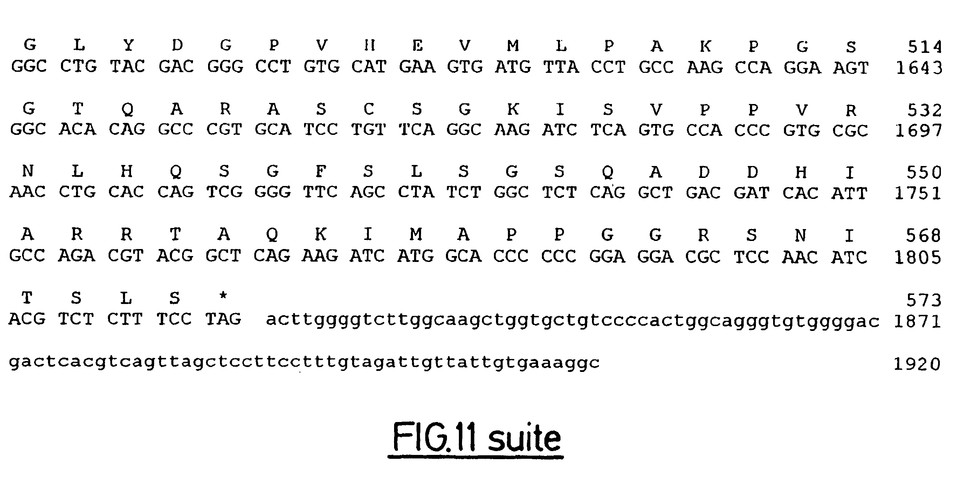

- La figure 11 représente la séquence nucléotidique d'ULIP-4 chez la souris (SEQ ID N° 5), ainsi que la séquence en acides aminés déduite (SEQ ID N° 6).

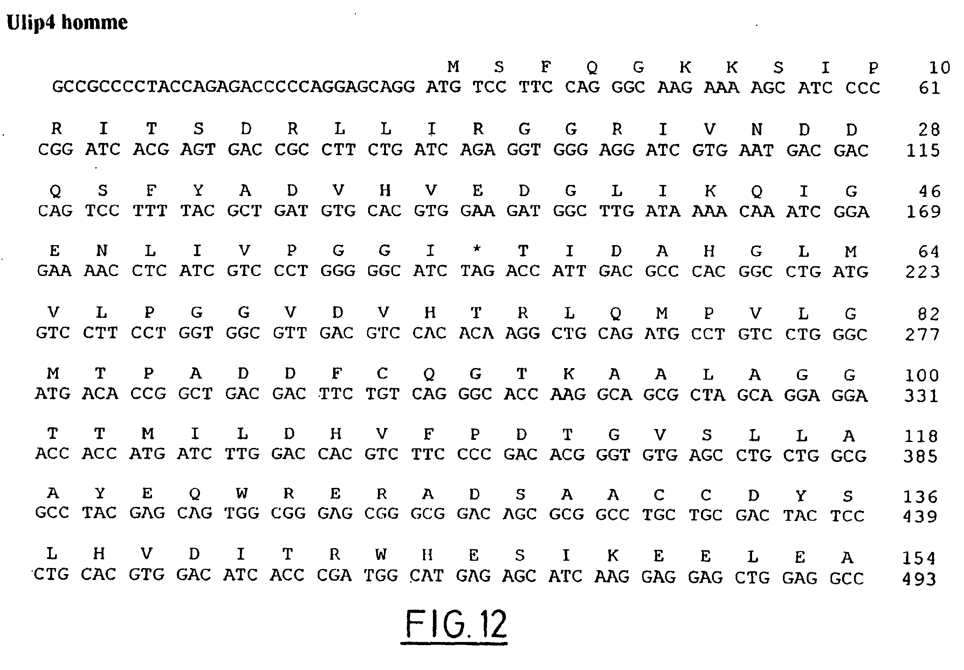

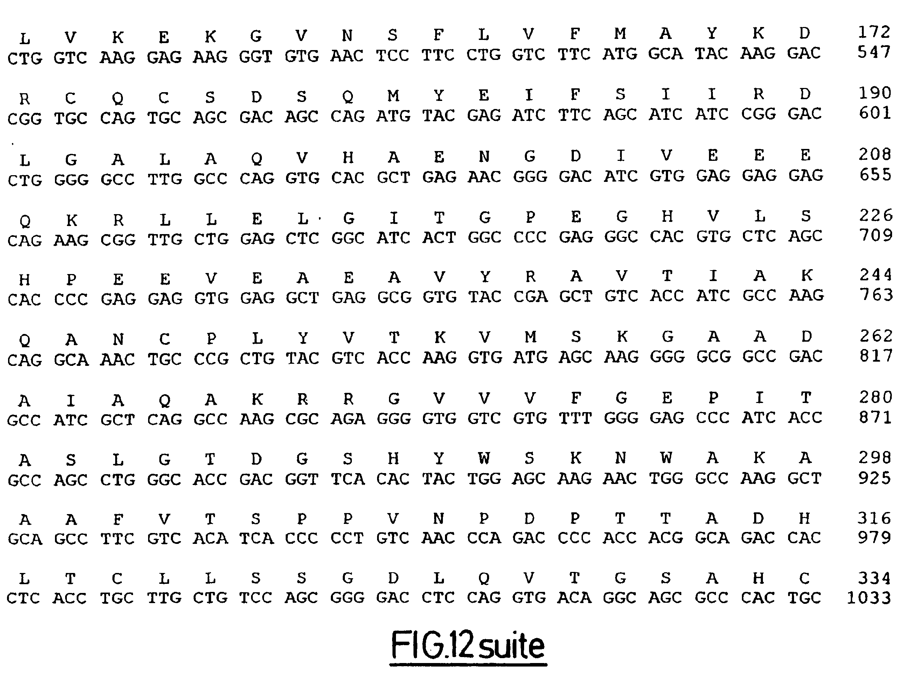

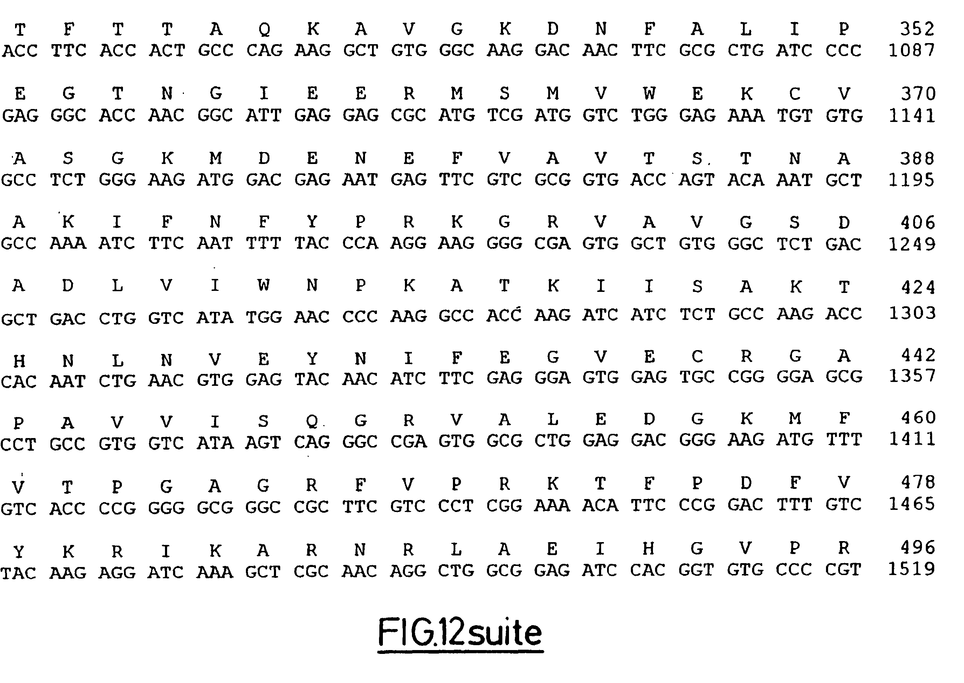

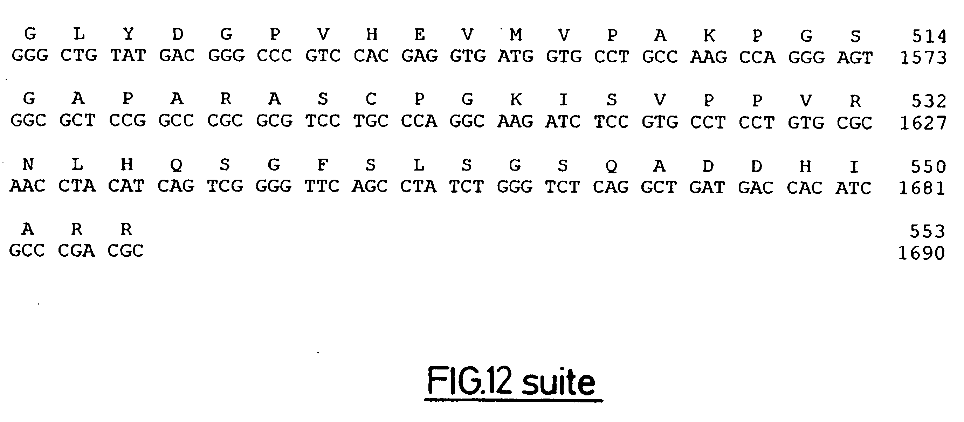

- La figure 12 représente la séquence nucléotidique d'ULIP-4 chez l'homme (SEQ 10 N° 7), ainsi que la séquence en acides aminés déduite (SEQ ID N° 8).

Un codon stop erroné dans la séquence ULIP-4 homme (astérisque) provient d'une faute de la transcriptase inverse dans la production de la banque. En comparant avec ULIP-4 de rat et de souris, il est presque certain que la séquence TAG codant pour un stop soit en fait un codon AAG, codant pour une lysine comme chez le rat et la souris. De plus, la région autour de cet acide aminé est entièrement conservée dans les trois espèces.

La séquence d'acides aminés a été complétée sur la SEQ ID n° 8 par 15 acides aminés en C-terminal (n° 554 à n° 568). Cette région C-terminale manquante sur la figure 12 est très bien conservée entre ULIP-4 rat et souris ainsi qu'entre les différentes ULIP.

### EXEMPLE 1 :

### Purification de POP-66 et séquençage

La purification de POP-66 est réalisée selon le matériel et les méthodes décrits dans l'article de Honnorat et al., 1996, à partir de sérum de patients atteints de SNP.

Pour identifier la protéine POP-66, on a choisi une stratégie de purification qui permette d'obtenir un séquençage partiel. Le criblage d'une banque d'expression d'ADNc de cerveau ou la purification de la protéine par immunoaffinité étaient exclus en raison des quantités limitées de sérums liées au décès des patients. Une méthode de purification biochimique a pu être développée à partir de cerveaux de rats nouveaux-nés grâce aux sérums humains anti-CV2 qui ont permis de suivre chaque étape de purification.

Les tissus, conservés à -70° C avant utilisation, ont été traités par une solution contenant DTT (dithiothreitol) (Sigma) 0,2 M. Ampholine 3-10 (Pharmacia) 2 %, Triton X-100 (Merck) 2 % et placés à 2-4° C. Immédiatement avant l'utilisation, de l'urée solide (Pharmacia) a été ajoutée pour obtenir une solution 8M.

La protéine POP-66 est soluble, au moins en partie, et précipite entièrement à une concentration de 40 % de sulfate d'ammonium.

Une centrifugation à 100 000 (fois) g et une précipitation au sulfate d'ammonium (éliminant les protéines précipitant en-dessous de 20 % et au-dessus de 40 % de sulfate d'ammonium) permettent d'obtenir des extraits protéiques enrichis en POP-66. Les protéines de cet extrait sont alors séparées, après dialyse, par isofocalisation sur gel d'agarose (Peltre et al., 1982).

Après transfert sur membrane, les anticorps anti-CV2 reconnaissent plusieurs bandes de points isoélectriques compris entre 5,85 et 6,55. L'ensemble de ces bandes correspond à la protéine POP-66 reconnue par les anticorps anti-CV2. Ce spectre suggère la possibilité de modifications transcriptionnelles (phosphorylations et/ou glycosylations) de la protéine.

A partir du gel d'agarose, la zone des protéines comprise entre 5,85 et 6,55, de pl, est utilisée pour une nouvelle migration électrophorétique en milieu dénaturant sur gel de polyacrylamide préalablement équilibré avec une solution d'équilibration (0,05 mol/l Tris/HCl, pH 6,8, urée 6M, glycérol 30 %, SDS 1 % poids/volume pendant 2 x 10 minutes) à laquelle on ajoute du DTT (0,25 % poids /volume) et du bleu de bromophénol.

Deux modes de détection sont utilisés :
- *la coloration à l'argent.* Immédiatement après la fin de la migration, le gel est immergé dans une solution fixatrice (40 % d'éthanol, 10 % d'acide acétique) pendant 30 minutes ; il est ensuite placé dans une solution d'incubation (30 % d'éthanol, 7 % poids/volume d'acétate de sodium, 0,1 % de glutaraldéhyde, 0,2 % poids/volume de thiosulfate de sodium) pendant 30 minutes ou une nuit. Après lavage, le gel est placé dans une solution d'argent (0,1 % poids/volume de nitrate d'argent + formaldéhyde) et développé (2,5 % poids/volume de carbonate de sodium + formaldéhyde). La réaction est arrêtée avec l'EDTA-Na₂ (1,5 % poids/volume). Les gels sont conservés dans une solution de glycérol.
- *transfert sur membrane de PVDF* (Immobilon-P®, Millipore). Les protéines séparées sont transférées sur une membrane de PVDF en utilisant un tampon CAPS (Sigma) 100 mM pH 11. Les transferts sont incubés pendant une heure dans du tampon TBS (Tris buffer saline) avec 5 % de caséine (lait) et 18 heures dans du tampon TBS (+ 1 % de caséine) contenant de l'anticorps (sérum anti-CV2 1/500). Après lavage avec TBS-caséine (15 minutes), la révélation est effectuée en incubant les transferts pendant 1 heure et demie avec les anticorps anti-IgG biotinylés (1/1000) et pendant 1 heure et demie avec le complexe streptavidine-peroxydase (1/2000). Les transferts sont ensuite révélés avec du DAB (diaminobenzidine 0,06 % poids /volume dans Tris 0,05 M) et avec H₂O₂ (0,02 µg/ml).

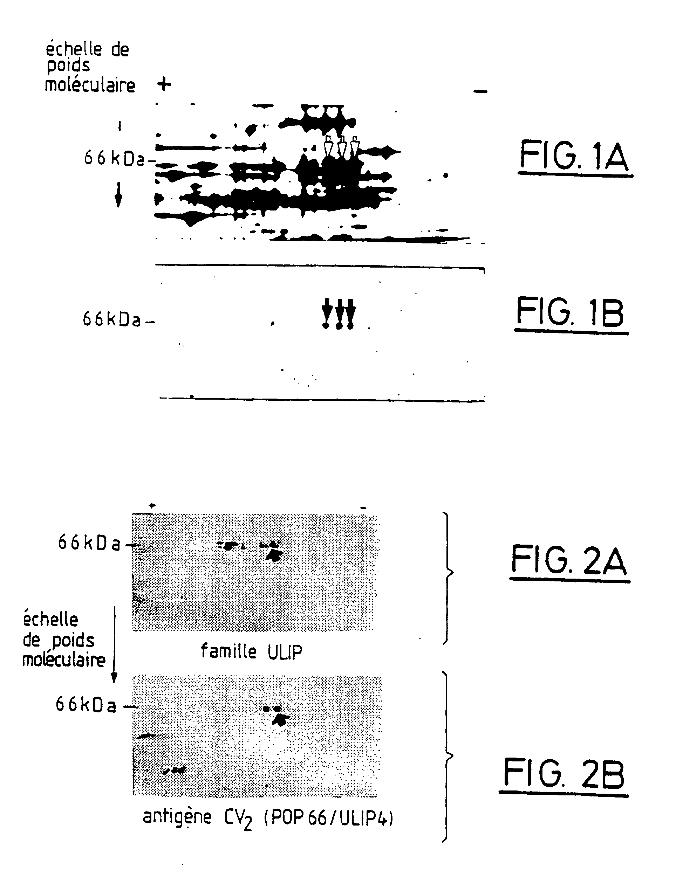

Une seule bande correspondant à une protéine de 66 kDa est visible. Celle-ci est spécifiquement marquée par les anticorps anti-CV2 (figure 1). On a effectué alors un séquençage N-terminal de cette protéine, après digestion trypsique.

Sept peptides, présentant les séquences suivantes, ont été obtenus :
1 - X-Met-Tyr-Asp-Gly-Pro
2 - X-Phe-Asn-Leu-Tyr-Pro-Arg
3 - X-Val-Leu-Glu-Asp-Gly-Thr-Leu-His-Val-Thr-Glu-Gly
4- X-Ile-Gly-X-X-Ala-Gln-Val-(His ?)-Ala-Glu-Asn-Gly-X-Ile-Ile-Ala-Glu-Glu-Gln
5 - X-X-Glu-Asn-Gln-Phe-Val-Ala-Val-Thr
6 - X-Val-Asn-Asp-(Asp ?)-Gln-Ser-Phe-Tyr-Ala-Asp-Ile-Tyr-Met-Glu-(Asp ?)-(Gly ?)-Leu-Ile
7 - X-X-X-Phe-Val-Thr-X-Pro-X-Leu-X-Pro
X : correspond à un acide aminé non déterminé,
(?) : correspond à un acide aminé probable mais non certain.

D'après l'analyse des banques de données disponibles en 1994, aucune protéine connue ne correspondait à ces séquences.

### EXEMPLE 2 :

### Clonage de l'ADNc de POP-66 ou des protéines apparentées

Le clonage de l'ADNc de la protéine POP-66 ou des protéines apparentées a été entrepris en utilisant des sondes oligonucléotidiques dégénérées obtenues à partir de fragments de deux peptides :
Ile-Ile-Ala-Glu-Glu-Gln
Tyr-Ala-Asp-IIe-Tyr-Met-Glu-(Asp ?)

Quatre jeux d'amorces oligonucléotidiques dégénérées (sens/anti-sens) sont donc déterminés
(AT(C/T)ATTGC(T/A)GA(A/G)CA;TG(C/T)TC(T/C)AC(T/A)GCAT(A/G)AT; TATGC(A/T)GA(CIT)AT(CIT)ATGGA ;TCCAT(G/A)TA(G/A)CT(T/A)GCAT A) et utilisés pour une amplification PCR.

La matrice est préparée sous forme d'ADNc double-brin (Promega kit) à partir d'ARNpoly(A⁺) extrait du cerveau de rats âgés de 10 jours (Zivic-Miller, USA) en utilisant le kit d'isolement de l'ARNm Fast Track (Invitrogen).

Les conditions d'amplification par PCR sont les suivantes : 35 cycles à 94°C, 1 minute pour la dénaturation, 55°C, 1 minute pour l'hybridation et 72°C, deux minutes pour l'extension.

Les produits de PCR sont analysés en électrophorèse sur gel d'agarose à 1 %, électroélués, clonés dans un vecteur de clonage TA (Invitrogen) et séquencés en utilisant les sites des amorces des promoteurs T7 et SP6.

La séquence d'acides aminés déduite du clone MFB-17 concorde avec les séquences des deux peptides originaux de POP-66 déterminés par l'analyse de la séquence d'acides aminés.

Une analyse comparée des séquences d'acides nucléiques utilisant les bases de données Genbank et EMBL révèle que MFB-17 est un ADNc partiel avec une séquence nucléotidique identique à celle d'un segment de TOAD-64, une protéine neuronale de rat (Minturn et al., 1995).

La séquence d'acides aminés déduite à l'ADNc de TOAD-64 concorde avec les séquences des sept peptides déterminés par l'analyse des séquence partielles de la protéine reconnue par les anticorps anti-CV2 après purification par électrophorèse.

Le poids moléculaire, le point isoélectrique, le profil immunohistochimique et la régulation de TOAD-64 sont similaires à ceux de l'antigène POP-66.

Le clone MFB-17 ne présentant pas la région codante complète, il a été nécessaire de produire une protéine recombinante intacte pour poursuivre les recherches concernant la protéine CV2.

Pour obtenir une protéine complète TOAD-64, on a amplifié la matrice ADNc-ds de cerveaux de rats avec deux jeux d'amorces situées aux extrémités 5' et 3' des régions codantes
(sens : GGCATATGTCTTATCAGGGGAAG ;
anti-sens GCGAATTCTTAGCCCAGGCTGATG).

Cette approche a permis de produire deux clones différents, l'un correspondant à la séquence TOAD-64 et l'autre à un clone désigné par C-22.

### EXEMPLE 3 :

### Comparaison de la séquence d'acides aminés déduite de C-22 avec les protéines ULIP

La séquence d'acides aminés déduite à partir du cadre de lecture ouverte, indique que ce clone C-22 appartient à la super-famille de gènes ULIP représentée par plusieurs gènes des séquences EST.

La séquence d'acides aminés déduite de C-22 présente une homologie de 30 % avec la séquence d'acides aminés de la protéine unc-33 de *Caenorhabditis elegans.*

Récemment, quatre gènes différents homologues à la protéine unc-33 ont été décrits chez les mammifères et le poulet.

Une analyse des séquences par les bases de données Genbank et banques de protéines a permis de proposer une classification des protéines unc-33 like (ULIP) en quatre différents sous-groupes (Byk et al. 1996).

Pourtant, comme les fonctions réelles de ces protéines ne sont pas clairement connues, la classification proposée est basée simplement sur le pourcentage d'identité d'acides aminés. ULIP-1 est représentée par une phospho-protéine « unc-33 like » de souris qui présente une homologie de 76 % avec TOAD-64, Crmp-62, et Munc, une séquence de souris récemment disponible sur Genbank.

ULlP-2 est composée de TOAD-64, Crmp-62 et Munc qui présentent entre eux une identité de 97 % d'acides aminés.

Les séquences partielles humaines EST, c'est-à-dire hcrmp-1, qui présentent une identité de 75 % avec ULIP-1 ou ULIP-2 ont été trouvées. Elles appartiennent à un troisième groupe appelé ULIP-3. Le dernier groupe identifié appelé ULIP-4, comprend r-CRMP-3 chez le rat et les formes ULIP-4 chez la souris et POP-66/ULIP-4 chez l'homme.

La comparaison de la séquence d'acides aminés des trois gènes ULIP, à savoir TOAD-64 chez le rat, Crmp-62 chez le poulet, et ULIP-1 chez la souris, avec la séquence d'acides aminés déduite du cadre de lecture ouvert du présent clone C-22, en utilisant le logiciel d'alignement Clustal V, révèle que C-22 présente une identité de 74% avec ULIP-1, 77 % avec Crmp-62 et 76 % avec TOAD-64.

La séquence nucléotidique C-22 a une identité de 97 % avec la séquence partielle EST, hCrmp-1, et définit ainsi le troisième membre du groupe ULIP-3. Les gènes TOAD-64, Crmp-62 et C-22 codent chacun pour une protéine de 572 acides aminés de longueur, tandis que la séquence d'acides aminés déduite à partir d'ULIP-1 donne une protéine de 570 acides aminés.

L'analyse de la séquence d'acides aminés de C-22 ne montre aucune séquence de signal ou de domaine transmembranaire suggérant que le ou les produits du gène C-22 pourraient être localisés dans le cytoplasme des cellules.

Plusieurs sites consensus de phosphorylation par la protéine kinase C (S/T X R/K) apparaissent le long du produit du gène C-22. Ces observations suggèrent que C-22 est une phospho- protéine et que de légères différences dans la phosphorylation pourraient dicter l'activité ou le rôle des différents membres de cette famille de protéines tout au long du cycle cellulaire.

**Tableau I : Récapitulation des protéines présentant une homologie avec les ULIP.**

| Famille | | Espèces | N° EMBL |
|---|---|---|---|
| Unc-33 nematode | | Nematode | Z14146 |
| | | | |
| Dihydropyrimidinase | Hu DHPase | humain | D78011 |
| | Ra DHPase | rat | D63704 |
| | | | |
| groupe ULIP-1 | Ulip | souris | X87817 |
| | Hu DRP3 | humain | D78014 |
| | r-CRMP-1 | rat | U52102 |
| | Hu-Ulip | humain | Y07818 |
| | | | |
| groupe ULIP-2 | ULIP-2 | souris | SEQ ID n° 2 |
| | Toad-64 | rat | Z46882 |
| | CRMP-62 | poulet | U17277 |
| | Munc | souris | X87242 |
| | HCRMP-2 | humain | U17279 |
| | Hu DRP-2 | humain | D78013 |
| | r-CRMP-4 | rat | U52104 |
| | | | |
| groupe ULIP-3 | ULIP-3 | souris | SEQ ID n° 4 |
| | HCRMP-1 | humain | U17278 |
| | rCRMP-1 | rat | U52102 |
| | C-22 | rat . | U52095 |
| | Hu DRP-1 | humain | D78012 |
| | | | |
| groupe ULIP-4 | ULIP-4 | souris | SEO ID n° 6 |
| | POP-66/ULIP-4 | homme | SEQ ID n° 8 |
| | r-CRMP-3 | rat | U52103 |

### EXEMPLE 4 :

### Régulation de l'expression du gène C-22 :

L'évaluation des altérations dans l'expression du gène C-22 pourrait avoir une importance considérable pour la connaissance des aspects fonctionnels de la protéine C-22.

Par conséquent, la Demanderesse a étudié la possible régulation de l'expression du gène C-22 au cours du développement. L'ARN total est extrait et séparé par l'électrophorèse sur gel d'agarose à 1 % et transféré sur membrane Nytran (Duchemin et al. 1987). Les transferts sont hybridés avec une séquence codante C-22 marquée au ³²⁻P; un tampon phosphate 0,5 mM et 5 % de SDS à 65°C pendant 16 heures.

A la fin de l'hybridation, les transferts sont lavés successivement trois fois avec 2xSSC, 0,1 % SDS à température ambiante, puis 1xSSC, 0,1 % SDS à 65°C pendant 60 minutes, et exposés aux rayons X.

Dans les conditions utilisées, on a détecté une seule bande à 3,8 kb représentant l'ARNm C-22 qui est aussi le plus petit transcrit de la famille de gènes unc-33 des vertébrés. La taille du transcrit reste la même durant les périodes pré et post-natales.

La cinétique du gène C-22 dans le cerveau de rats au cours du développement montre que le messager est détectable au cours de la période embryonnaire au jour E17. La quantité de transcrits C-22 augmente jusqu'au jour 7 post-natal puis décroît rapidement à partir de la seconde semaine après la naissance jusqu'à un niveau pratiquement indétectable chez l'adulte.

Aux environs de la naissance, un signal de régulation encore inconnu est probablement reçu, ce qui augmente l'expression du gène C-22, ce signal étant lié temporairement à la différenciation neuronale et au développement axonal.

L'ARNm de C-22 n'a pas pu être détecté par analyse Northern Blot dans plusieurs régions du cerveau telles que le cortex frontal , le cerveau moyen et le thalamus chez l'adulte et le rat âgé de plus de deux ans.

De plus, on n'a pas pu détecter l'ARNm de C-22 dans des tissus non neuronaux, tels que le coeur, le poumon, le foie, le rein chez des rats d'une semaine et des rats adultes.

Les données sur le profil de l'expression l'ARNm de C-22 suggèrent un rôle prépondérant de la protéine C-22 dans le développement du cerveau.

### EXEMPLE 5 :

### Immunoempreinte de POP-66 :

### A - Matériels et méthodes

### • Transfection des ULIP dans E. coli

Les ADNc de pleine longueur d'ULIP-2 et ULIP-3 de rat et ULIP-1 et ULIP-4 de souris ont été sous-clonées de manière directionnelle dans le vecteur d'expression pET-21a(+) *E. Coli* après introduction d'un site 5'Nde I et d'un site 3' EcoRI par PCR, et les quatre constructions ont été reséquencées. L'expression de gène cible induit par IPTG a été réalisée selon le protocole du fabricant (Novagen).

### • Production d'anticorps anti-ULIP

Des anticorps de lapin (anti-Pep3) sont dirigés contre le peptide ITGPEGHVLSRPEEVE (acides aminés 217-232 de la séquence SEQ ID n° 8), synthétisé sur un appareil de synthèse peptidique multiple utilisant le F-moc (432A Peptide Synthesizer SYNERGY, Applied Biosystems). La pureté a été vérifiée par analyse de la séquence par HPLC et spectrométrie de masse. 1 mg du peptide synthétique conjugué à de l'hémocyanine de patelle, dans de l'adjuvant complet de Freund, a été utilisé pour immuniser des lapins avec une dose « booster » de 0,5 mg de peptide lié dans de l'adjuvant complet de Freund après 4 semaines. Les anticorps anti-Pep3 ont reconnu les quatre protéines ULIP recombinantes exprimées dans *E. Coli.*

Le marquage avec les anticorps anti-Pep3 a été éliminé après préincubation avec le peptide 3. Des contrôles avec des sérums de lapin pré-immuns étaient négatifs.

Des anticorps anti-peptide 4 dirigés contre le peptide LEDGTLHVTEGS ont été produits selon le même protocole.

### B - Résultats

Des anticorps contre quatre des peptides séquencés ont été produits. Deux des sérums se sont avérés particulièrement intéressants.

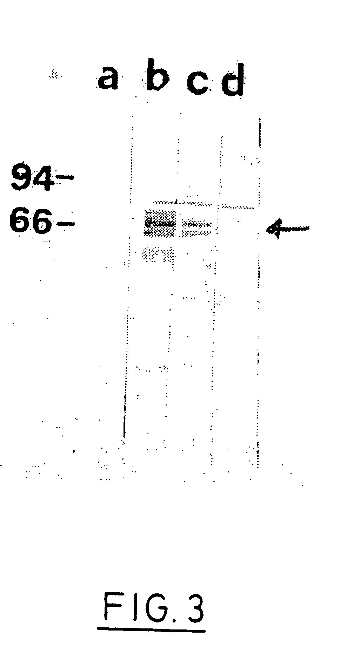

L'un contient des anticorps (Ac anti-pep3) qui reconnaissent plusieurs membres de la famille ULIP en électrophorèse à deux dimensions d'extraits protéiques de cerveau de rat nouveau né (figure 2) et sur électrophorèse à une dimension (figure 3). Un autre anticorps (Ac anti-pep4) reconnaît en Western Blot une seule bande de 66 kDa susceptible de correspondre à un seul membre de la famille (figure 3). à savoir ULIP-2.

### EXEMPLE 6 :

### Immunohistochimie

Les préparations de tissus pour l'immunohistochimie sont obtenues à partir de cerveaux de rats nouveaux-nés et de cerveaux humains *post-mortem,* fixés à 4° C dans du paraformaldéhyde à 4 % et 0,2 % d'acide picrique dilués dans du tampon phosphate (0,1 M, pH =7,4), puis cryoprotégés.

L'immunocytochimie peut être réalisée par la technique d'immunofluorescence indirecte. Des coupes de 12 µm d'épaisseur sont réalisées au cryostat puis montées sur lame recouverte de gélatine, traitées pendant 2 heures dans du tampon PBS et 1 % de sérumalbumine bovine (BSA) avec 0,1 % de Triton X100 et incubées 12 h avec le sérum de patients anti-CV2 dans du PBS-1% BSA à température ambiante (dilution du sérum 1/100). Après plusieurs lavages avec PBS-1 % BSA, les coupes sont incubées pendant 2 h avec un anti-sérum anti-humain de lapin conjugué à la fluorescéine diluée à 1 % (Dakopatts) dans du PBS-1 % BSA. Après lavage dans le PBS les lames sont examinées au microscope. Les coupes contrôle sont incubées avec soit l'antisérum IgG anti-humain conjugué à la fluorescéine seule, soit le PBS-1 % BSA seul, soit le sérum de patient seul, soit enfin le sérum contrôle (patients non atteint de SNP) et anticorps conjugué à la fluorescéine à la même dilution.

Pour confirmer l'immunofluorescence on peut utiliser le marquage indirect par immunoperoxydase. Les coupes de tissus congelés fixés au paraformaldéhyde sont incubées avec 0,3 % d'H₂O₂ (pour détruire l'activité peroxydase intrinsèque) et 10 % de sérum normal de lapin (pour éviter la liaison non spécifique des IgG de lapin) ou 1 % BSA. Après incubation 12 h avec des sérums de patients dilués à 111000 et lavage, les coupes sont incubées 2 h avec de l'antisérum IgG anti-humain de lapin biotinylé dilué à 1/1000 dans PBS-1% BSA. Les IgG humains liés sont visualisés par incubation avec un complexe avidine-biotine-peroxydase (Vectastain ABC complex, Vector) et développés avec 0,05 % DAB (Sigma). Les coupes contrôle sont obtenues avec des sérums de 15 patients sans SNP selon le même protocole.

### A - Localisation des protéines de la famille ULIP à l'aide d'anticorps antipeptides :

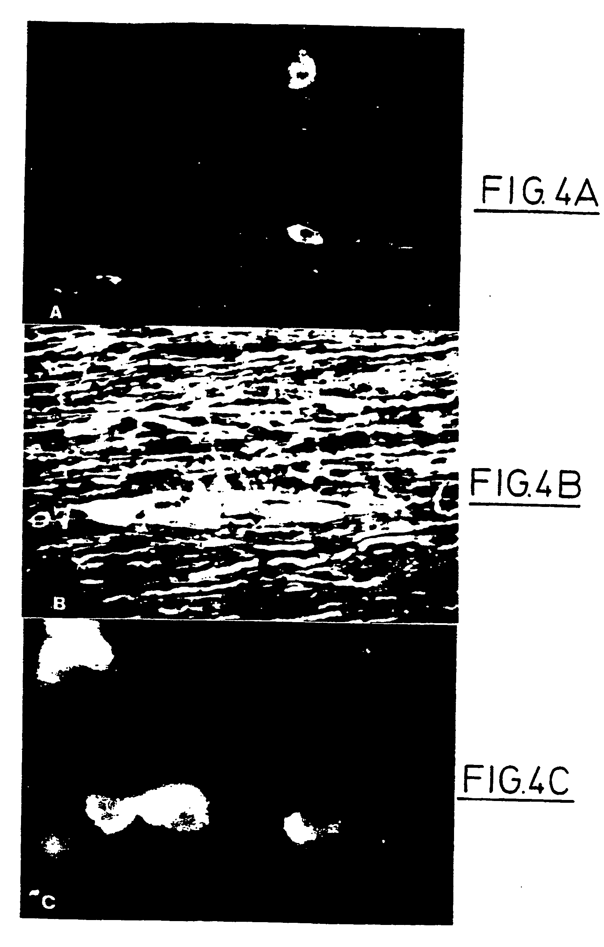

Un marquage immunohistochimique a été réalisé sur coupe de cerveaux de rats nouveaux-nés et adultes. L'anticorps antipeptide-3 reconnaît un (des) antigènes présent(s) dans plusieurs types cellulaires sur coupe de cerveaux de rats nouveau-nés et adultes (fig. 4). Comme le sérum anti-CV2 de patient, les anticorps anti-peptide-4 ne permettent la mise en évidence d'aucun antigène sur coupe de cerveau de rat nouveau né alors qu'ils marquent spécifiquement une sous-population d'oligodendrocytes dans le cerveau de rat adulte (fig. 4).

### B - Expression de POP-66 au cours du développement normal du cerveau :

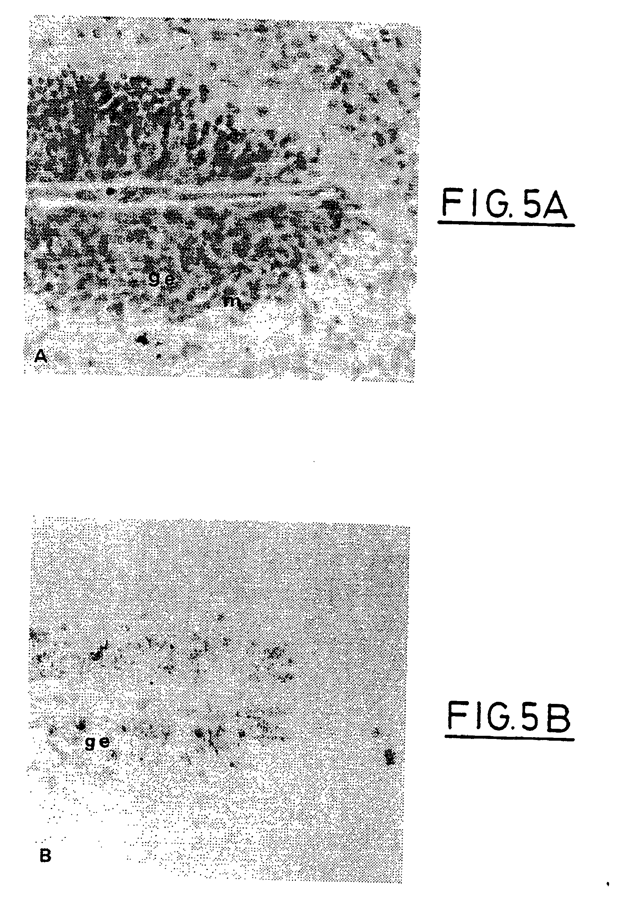

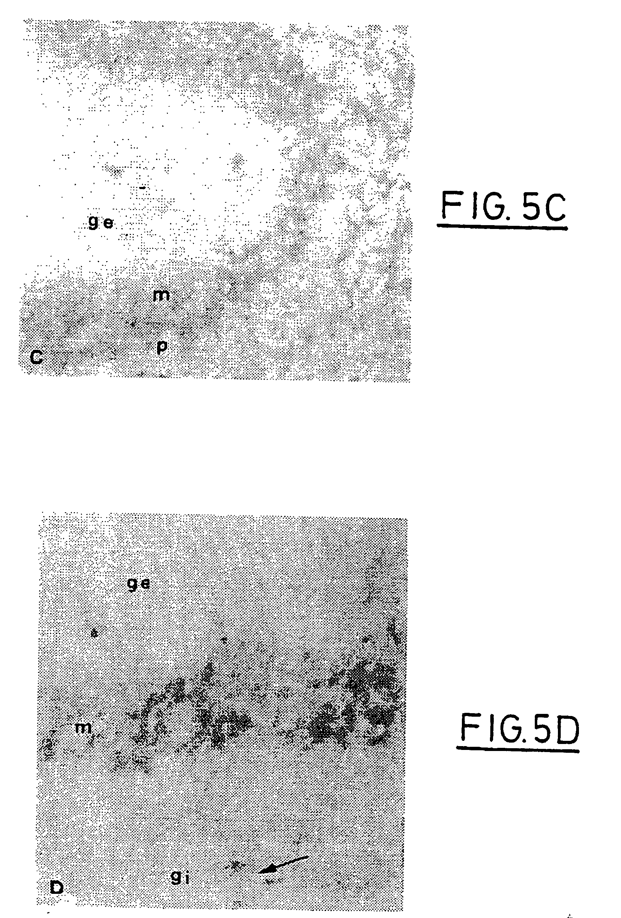

La figure 5 montre que les cellules nerveuses prolifératives des zones progénitrices du système nerveux mises en évidence par l'accumulation de bromodéoxyuridine (BrdU) n'expriment pas POP-66 alors que les cellules non prolifératives qui correspondent aux cellules nerveuses en différenciation ou en migration l'expriment.

### EXEMPLE 7 :

### Rôle de POP-66 dans la survie neuronale

La figure 6 permet de comparer des coupes de cerveaux humains de patients sains et de patients atteints de SNP. Chez les patients atteints d'un SNP et présentant des anticorps circulant anti-CV2, on observe une disparition des neurones du gyrus dentatus et des neurones pyramidaux (bande de cellules centrale), ainsi qu'une réaction astrocytaire intense.

### EXEMPLE 8 :

### Caractérisation de la protéine POP-66 - Identification avec ULIP-4 :

### Matériels et méthodes

### a) Purification partielle d'ULIP-1

ULIP-1 partiellement purifiée a été obtenue à partir de cerveaux de souris nouveaux-nés par trois étapes de purification. Ces cerveaux ont été homogénéisés dans 4 volumes de tampon d'homogénéisation (25 mM phosphate de sodium, pH 7,8, 1 mM EGTA, 10 µg/ml de leupeptine, 25 µg/ml d'aprotinine, et 10 µg/ml de pepstaine. Les homogénats ont été centrifugés pendant 10 minutes à 400 x g. Les culots ont été resuspendus dans 2 volumes de tampon d'homogénéisation, homogénéisés, et de nouveau centrifugés. Les surnageants issus des deux centrifugations ont été rassemblés, soniqués et centrifugés pendant 1 heure à 100.000 x g. Le surnageant (S2) a été chargé sur une colonne CL-6B de DEAE-Sepharose (1,75 cm² x 26 cm) équilibré avec 100 ml de tampon A (25 mM de phosphate de sodium, pH 7,8, 1 mM d'EGTA) à une flux de 30 ml par heure. Les protéines ont été éluées dans 300 ml d'un gradient linéaire de chlorure de sodium 0-250 mM dans du tampon A et des échantillons de 5 ml ont été recueillis. Les fractions contenant ULIP ont été collectées et du sulfate d'ammonium solide a été ajouté jusqu'à 20 % de saturation. Ce « pool » a été chargé sur une colonne CL-48 de phényl-Sepharose (1,75 cm² x 22 cm) qui a été préalablement équilibré avec 100 ml de tampon B (10 mM de phosphate de sodium, pH 7,8, 1 mM d'EGTA) contenant 20 % de sulfate d'ammonium saturé. Les protéines ont été éluées dans un gradient linéaire décroissant de 20 à 0 % de sulfate d'ammonium saturé dans du tampon B. Les fractions contenant ULIP ont été recueillies, dialysées deux fois contre 20 volumes de tampon A. Les protéines ont été concentrées sur une petite (10 ml) colonne C1-6B de DEAE-Sepharose et éluées avec 400 mM de chlorure de sodium dans du tampon A. L'éluat a été dessalé sur une colonne Sephadex G-25 (NAP-10) et concentré dans un volume final de 0,5 ml par évaporation. Dans la dernière étape de purification, la fraction concentrée a été chromatographiée en trois étapes successives, sur deux colonnes de FPLC (Fast Protein Liquid Chromotagraphy) Superose 12 montées en série, dans du tampon C (50 mM de phosphate de sodium, pH 7,2, 150 mM de chlorure de sodium) à un débit de 0,3 ml/minute. Les fractions (0,6 ml) ont été recueillies et les fractions enrichies en ULIP ont été analysées. La présence d'ULIP dans les étapes successives de purification a été testée par un Western Blot à une dimension en utilisant un anticorps anti-stathmine capable de réactivité croisée. Les protéines ont été quantifiées selon la méthode de Bradford.

### b) Migration sur net d'électrophorèse :

Une électrophorèse à une dimension a été effectuée sur des gels de polyacrylamide 13 % selon la méthode de Laemmli. Les électrophorèses PAGE à deux dimensions ont été effectuées tel que décrit précédemment. Les gels d'isoélectrofocalisation contenaient 2 % d'ampholines total, pH 6-8 et 3-10 selon un rapport de 4 :1. La seconde dimension avait été menée sur des gels d'acrylamide à 10 %. Les protéines ont été soit soumises à une immuno-empreinte soit colorées par l'argent.

### c) Analyse Western Blot :

Les protéines ont été transférées à partir des gels sur de la nitrocellulose dans du tampon contenant Tris 48 mM, glycine 39 mM et 5% de méthanol. La membrane a été saturée avec de la caséine (2,5 %) dans la solution d'immuno-empreinte (12 mM de Tris-HCl, pH 7,4, 160 mM de Na Cl, 0,1 % de Triton X-100) et testée avec un antisérum dirigé contre le peptide I de la stathmine de rat (dilution 1/10.000) ou un antisérum dirigé contre la protéine ULIP recombinante (dilution 1/20.000) dilué dans une solution d'immuno-empreinte contenant 1 % de caséine. Les anticorps liés ont été détectés soit avec une protéine A marquée à |^{'125}| et autoradiographiés soit avec des anticorps anti-lapin liés à la peroxydase en utilisant le kit ECL (Amersham).

### d) Analyse de la séquence protéique :

Les fractions enrichies en ULIP ont été séparées sur les gels de polyacrylamide à deux dimensions. Les gels sont fixés pendant 30 minutes dans 25 % d'éthanol et 10 % d'acide acétique et colorés pendant 3 minutes dans 0,1 % de noir amido dans 1 % d'acide acétique et 40 % de méthanol. Les gels ont été décolorés dans 1 % d'acide acétique et les taches correspondant à la forme principale de ULIP ont été découpées dans ces trois gels, recueillies et digérées avec 2 mg/ml d'endoprotéase Lys C. Les peptides élués du gel ont été ensuite séparées par HPLC sur colonne DEAE-C18 avec un gradient de 0-55 % d'acétonitrile dans 0,1 % d'acide trifluoroacétique. Les peptides ont ensuite été séquencés selon la dégradation automatique d'Edman.

### e) Expression in vitro chez un mammifère

1 µg du plasmide Bluescript contenant l'AONc entier codant pour ULIP-1, ULIP-2, ULIP-3 ou ULIP-4 a été utilisé pour réaliser la transcription et la traduction *in vitro* avec le système « Reticulocyte lysate » (Promega) selon le protocole décrit par le fabricant. 5 µg du mélange de 25 µl total de transcription/traduction ont été analysés sur gel d'électrophorèse à deux dimensions.

### Résultats

Ni la protéine recombinante ULIP-1, ni les protéines recombinantes TOAD-64 (ULIP-2) et C-22 (ULIP-3) ne sont reconnues par les sérums anti-CV2. De plus, le profil de distribution des taches correspondant à POP-66 reconnues par les anticorps anti-CV2 en électrophorèse à deux dimensions ne correspond pas aux taches reconnues par les anticorps anti-UL1P-1. Or, POP-66 est un membre de la famille ULIP puisque les trois taches POP-66 sont reconnues par l'Ac anti-pep3. POP-66 correspond donc à un membre de la famille de pHi plus basique.

Après traduction *in vitro* des quatre protéines(ULIP-1, 2, 3, 4), on a montré que ULIP-4 a le même profil électrophorétique 2D que POP-66 et est reconnue par les anticorps anti-CV2 (figure 7).

Pour cela, la protéine ULIP-4 et, comme contrôle, la protéine ULIP-2 ont été traduites *in vitro* en présence de méthionine ³⁵S à partir de clones d'ADNc codants pour les protéines entières. Les protéines ont été séparées par électrophorèse à deux dimensions (en présence d'un extrait de cerveau fournissant les repères essentiels), transférées sur nitrocellulose et révélées :
- par autoradiographie pour localiser les protéines traduites *in vitro* (traduction) ;
- par immunoempreinte avec le sérum CV2.

La figure 7 montre que les trois taches de la traduction *in vitro* d'ULIP-4 correspondent aux taches reconnues par CV2. Ces taches ne sont pas reconnues dans la traduction d'ULIP-2.

Le sérum CV2 reconnaît donc spécifiquement ULIP-4.

Ceci a permis d'identifier POP-66 comme ULIP-4.

### EXEMPLE 9 :

### Localisation chromosomique de la protéine POP-66/ULIP-4

L'ADNc d'ULIP-4 humain étant cloné, il est alors possible de déterminer la localisation chromosomique du gène POP-66/ULIP-4 par cartographie génique par hybridation *in situ* isotopique (Levy et Mattei et al., 1995) .

L'hybridation *in situ* est menée sur des préparations de chromosomes obtenues à partir de lymphocytes humains stimulés par la phytohémagglutinine mis en culture pendant 72 heures. De la 5-bromodéoxyuridine a été ajoutée pendant les 7 dernières heures de la culture (60 µg/ml de milieu), pour assurer une image de bandes chromosomiques post-hybridation de bonne qualité. Le clone contenant un insert de 1300 paires de bases codant pour ULIP-4 dans le vecteur Bluescript est marqué au tritium par translation de coupure (« nick translation ») avec une activité spécifique de 1x10⁸ dpm. µg-1. La sonde radiomarquée a été hybridée au stade métaphase à une concentration finale de 200 ng par ml de solution d'hybridation. Après recouvrement par une émulsion Kodak NTB₂ les lames ont été exposées pendant 20 jours à +4°C puis développées. Pour éviter le décalage des grains d'argent pendant le processus, les étalements de chromosomes ont été préalablement marqués avec une solution tampon de Giemsa et les métaphases ont été photographiées. La révélation des bandes a été effectuée par la méthode « fluorochrome-photolyse Giemsa » (FPG) et les métaphases ont été rephotographiées avant analyse. Sur les 100 cellules en métaphase examinées après hybridation *in situ,* on a dénombré 246 grains d'argent associés aux chromosomes et 54 parmi ceux-ci (21.9 %) étaient localisés sur le chromosome 10. La distribution des grains sur ce chromosome n'était pas aléatoire : 39 sur 54 (72,2 % de ceux-ci) étaient localisés sur la région q25.2-q26 du bras long du chromosome 10.

Le gène POP-66/ULIP-4 se trouve donc situé sur le chromosome 10 dans la région q25.2-q26. Dans cette région chromosomique, les locus de maladies neurodégénératives et de gènes suppresseurs de tumeurs impliqués dans différents types de cancers ont été localisés. Le locus d'une maladie cérébelleuse à début précoce (« infantil onset spinocerebellar ataxia ») a été identifié dans la région 10q24-26 (Varilo et al., 1996 ; Nikati et al., 1995). Les symptômes de cette maladie dégénérative hériditaire récessive caractérisée par une ataxie, une neuropathie et une atrophie optique sont similaires à ceux observés chez les patients atteints de syndromes neurologiques paranéoplasiques avec des auto-anticorps anti-CV2 circulants (Honnorat et al., 1996). D'un autre côté, 80 % des glioblastomes présentent des mutations dans cette région chromosomique et plusieurs locus suppressifs impliqués dans différents types de tumeurs (prostate, rein, cancer du poumon à petites cellules et carcinomes de l'endomètre) sont localisés dans cette région chromosomique. Ces données renforcent la possibilité que PO66/ULIP-4 joue un rôle crucial dans la neurodégénération et la tumorigénèse.

A cet égard, il est notable que l'expression de ULIP-1 est régulée à la hausse dans des cellules de neuroblastomes différenciés par l'acide rétinoïque et que ULIP-1 et ULIP-3 sont régulés à la hausse mais ULIP-4 est régulé à la baisse dans des cellules PC12 différenciées en présence de NGF, suggérant que l'arrêt de la croissance cellulaire peut être lié à des niveaux d'expression des protéines ULIP.

### EXEMPLE 10 :

### Expression des protéines ULIP dans les cellules HeLa transfectées

### A - Matériels et méthodes

Une séquence « flag » (EcoRl-ATGGACTACAAGGACGACGATGACAAGG-BamHI) (Kodak) a été clonée dans le site EcoRI de pSG5 suivi par ULIP-1 (EMBEL X87817, paires de bases: 309-2023), Ulip2 (Y10339, paire de bases : 23-1741), Ulip3 (Y09080, paires de bases : 269-1991) ou Ulip 4 (Y09079, paires de bases : 102-1820), respectivement. Les cellules HeLa ont été cultivées dans des milieux DMEM (Gibco) additionnées de 10 % de sérum de veau foetal (v/v). Les transfections ont été réalisées par précipitation au phosphate de calcium (Maniatis et al., 1978). Les cellules HeLa ont été mélangées avec 5 µg de plasmides pSG5flag-ULIP-1, 2, 3, 4 et 10 µg de pUC18. Vingt quatre heures après la transfection, les cellules HeLa ont été fixées avec 4 % de paraformadehyde et immunomarquées avec différents sérums humains (dilution 1/300), révélées par des anticorps anti-IgG humains conjugués à la FITC (Biosys), ou des anticorps anti-fiag (M2. Kodak) (dilution 1/1000), révélés par des anticorps anti-lapin conjugués au rouge Texas (Vector).

Un double immuno-marquage a été réalisé sur les cellules HeLa transfectées par des ULIP en utilisant des anticorps anti-flag et anti-Pep3. Dans les cellules transfectées par un quelconque ADNc, 10 à 20 % d'entre elles présentaient un immuno-marquage avec les anticorps anti-flag révélés par les anticorps anti-souris conjugués au rouge Texas.

Toutes les cellules transfectées ont été marquées en double par des anticorps dirigés contre Pep3, un peptide commun aux quatre ULIP est révélé par des anticorps anti-IgG de lapin conjugué à la fluoresceine.

Un double immuno-marquage a également été réalisé sur les cellules HeLa transfectées par des ULIP en utilisant des anticorps anti-flag et anti-CV2. Les sérums humains de patients atteints de SNP avec des auto-anticorps circulant anti-CV2 ont marqué les cellules transfectées par ULIP-4, et un sérum anti-CV2 a également marqué les cellules transfectées par ULIP-3. Aucun marquage des cellules transfectées par ULIP-4 n'a été détecté dans les sérums contrôles de patients sans cancer ou maladie neurologique.

### B ) Résultats

Après transfection des cellules HeLa avec des ADNc marqués par les flag des 4 ULIP, 10 à 20 % des cellules étaient fortement réactives avec des anticorps anti-flag et des anticorps anti-Pep3 qui reconnaissent les 4 ULIP de mammifères. Les cellules transfectées n'ont pas été immuno-marquées avec du sérum contrôle de 10 patients neurologiques sans SNP ni avec du sérum pré-immun de lapin. En revanche, les cellules transfectées avec de l'ADNc d'ULIP-4 ont montré une immuno-réactivité intense avec tous les 7 sérums testés de patients avec des auto-anticorps anti-CV2 circulants. Ces sérums sont négatifs sur des cellules transfectées avec des ADNc d'autres ULIP, à l'exception d'un sérum qui a également reconnu les cellules transfectées par ULIP-3 et un sérum qui a également reconnu les cellules transfectées par ULIP-1, 3 et 4. Aucun marquage n'a été observé sur des cellules HeLA non transfectées, avec un sérum anti-CV2.

Le tableau I ci-après présente les résultats d'immunofluorescence indirecte avec différents sérums sur les cellules HeLa par des ADNc marqués de membres de la famille de ULIP.

**Table 1 :**

| N° sérum | Symptomes neurologiques | Type de tumeur | Ulip- 1 | Ulip-2 | Ulip-3 | Ulip-4 |
|---|---|---|---|---|---|---|
| Anti-Pep3 | - | - | + | + | + | + |
| pre-immun | - | - | - | - | - | - |
| Pep3 | | | | | | |
| 90-002 | PCD, uvéite | UC | - | - | + | + |
| 93-484 | LE | Thymome | - | - | - | + |
| 94-590 | LE | SCLC | - | - | - | + |
| 95-700 | PEM | SCLC | + | - | + | + |
| 95-701 | PCD | sarcome utérin | - | - | - | + |
| 95-706 | LE, neuropathie | SCLC | - | - | - | + |
| 97-040 | PCD | SCLC | - | - | - | + |
| 97-103 | PCD | SCLC | - | - | - | + |

PCD : dégénération paranéoplasique du cervelet ;
LE : encéphalite lymbique ;
PEM : encéphalomyélite paranéoplasique ;
UC : carcinome indifférencié ;
SCLC : carcinome de poumons à petites cellules.

### EXEMPLE 11 :

### Expression de POP-66/ULIP-4 et des membres de la famille ULIP dans les cancers

### A - Expression de ULIP-2 et ULIP-3 dans les cancers :

### 1) Matériels et méthodes : expériences de RT-PCR :

L'ARN total a été extrait en utilisant 1 ml de RNAZOL™B (Bioprobe) selon la méthode de Chomczynski et Sacchi. La quantité d'ARN a été déterminée par densité optique mesurée à 260 nm et sa pureté a été déterminée à partir du rapport des absorbances mesurées à 260 et 280 nm (rapports 1,8-2,0). L'intégrité des préparations d'ARN a été en outre vérifiée par électrophorèse sur gel d'agarose à 1 % dans du TBE (0,45 M de Tris-borate, 10 mM d'EDTA, pH 8): La spécificité des amorces a été analysée en comparant leurs séquences avec les diverses banques de données de gènes (EMBL et FASTA). Pour une quantification relative, le gène codant pour la G3PDH (glycéraldéhyde-3-phosphate déshydrogénase, Clontech), gène ubiquitaire exprimé dans de nombreux tissus incluant le cerveau, a été co-amplifié avec l'ARNm testé en tant que standard interne pour vérifier l'égalité des quantités d'ARN des échantillons et pour tester l'efficacité de l'étape de transcription inverse pour les différents échantillons d'ARN. Les amorces 5', 3' et les oligonucléotides des sondes internes de G3PDH ont été synthétisées et purifiées par Eurogentec. L'ARNm total (1 µg) a été dénaturé (15 minutes à 65° C) et transcrit en ADNc simple brin (1 heure et demie, 42° C) dans un volume final de 20 µl de tampon (50 mM de Tris-HCl, 75 mM de KCI, pH 8,3, Gibco BRL) contenant 5 ng par µl d'amorce oligo-dT 12-18 (Pharmacia Biotech), 40 unités de reverse transcriptase du virus de la leucémie murine Moloney (Mu-LV) (Gibco BRL), 40 unités de RNAsine (Promega), 10 mM DTT (Gibco BRL) et 0,5 mM de chacun des déoxynucléotides triphosphate (Promega). Les échantillons d'ADNc ont été dilués au 1 /10 dans de l'eau distillée et les réaction de PCR ont été menées en utilisant 1 µl, 4 µl ou 2 µl de l'échantillon d'ADNc pour l'ARN messager d'ULIP-2 et ULIP-3, dans un tampon (50 mM de KCl, 10 mM de Tris-HCl, 0,1 % de Triton X100, 0,4 % de glycérol et 800 µM de NaCl, Ph 9), dans lequel a été ajouté 40 µM de DTT, 3 mM de MgCl₂, 0,2 mM de chaque dNTP, 0,4 µM de chaque amorce sélectionnée et 2 unités de l'AmpliTaq ADN polymérase (Promega) dans un volume final de 50 µl. Les échantillons ont ensuite été placés dans un thermocycleur (Biomed-Hybaid), dénaturés à 95°C pendant 5 minutes et amplifiés pendant 35 cycles (un cycle = dénaturation 95° C pendant 65 secondes, hybridation des amorces 60° C pendant 45 secondes extension 72° C pendant 4 minutes et allongement finale 15 minutes à 72° C. Les produits ont été séparés par électrophorèse sur gel d'agarose-Seakem à 1 % et les bandes tests des produits de RT-PCR de taille attendue ainsi que l'échelle de marqueur de poids moléculaire (100 paires de base) (Promega) ont été visualisés en utilisant la coloration au bromure d'éthidium.

### Composition des sondes oligonucléotidiques utilisées pour la PCR ULIP-3 5' ATAGAGGAGCGGATGACG (899) 3'

GCTGTTATGGTCTTCAACTTGTCGG (1092)

GGCCTGTTATGGTCTTCAACTTGTCG (1093)

### Composition des sondes oligonucléotidiques utilisées pour la PCR ULIP-2 5' AGGAGGAGTGAAGACCATCG 5227) 3'

CTTATGCCACTCGCTGATGTCC (509).

### 2) Résultats

Les expériences de RT-PCR montrent que TOAD-64 (ULIP-2) et C-22 (ULIP-3) sont exprimés dans certaines tumeurs du poumon à petites cellules (cf. figure 8) et absents dans d'autres notamment dans celles des patients qui développent des syndromes neurologiques paranéoplasiques de meilleur pronostic.

### B - Expression de ULIP-4 dans les cancers

### 1) Matériels et méthodes

### • Préparation de l'ARN et RT-PCR

Les ARN totaux sont extraits de tumeurs cérébrales conservées dans l'azote liquide, selon la technique classique au RNAZOL™(Bioprobe, France). La transcription inverse a été effectuée en utilisant des oligo(dt)₁₈ sur 1 µg d'ARN total et la PCR a été réalisée avec 1/20 du volume du mélange pour la transcription inverse (RT-mix). Les amorces utilisées pour ULIP-4 sont :
5'CATCTGGCTGTCGCTGCAC3', 5'GCCGCCCCTACCAGAGACC3', et pour GAPDH : 5'GGAGATTCAGTGTGGTGG3', 5'GGCTCTCCAGAACATCATCC3'. L'ADNc a été dénaturé à 95°C pendant cinq minutes. L'amplification par PCR a été réalisée pendant 30 cycles. Ulip4 : 95°C, 45 sec ; 62°C, 45 sec ; 72°C, 45 sec. GAPDH : 95°C, 45 sec ; 55°C, 45 sec ; 72°C, 45 sec. L'extension finale a été réalisée à 72°C pendant 5 minutes.

### 2) Résultats

Sur les 8 extraits de glioblastomes étudiés, 4 (50 %) exprimaient l'ARN messager de ULIP-4. A l'inverse, sur les 10 extraits d'oligodendrogliomes testés, aucun n'exprimait l'ARN messager de ULIP-4. Cette expression différentielle, en fonction du type de tumeur cérébrale primitive, est en faveur d'un rôle potentiel de ULIP-4 dans la prolifération cellulaire de ces tumeurs.

La protéine POP-66/ULIP-4 ainsi que les protéines de la famille ULIP pourraient être exprimées dans les tumeurs périphériques (tumeur du poumon à petites cellules, thymome, cancer du sein et de l'ovaire). Leur présence pourrait donc être corrélée à un pronostic. La localisation du gène de POP-66/ULIP-4 sur la partie distale du chromosome 10 le confirme dans le cas des tumeurs cérébrales.

Ainsi, l'expression différentielle des membres de la famille ULIP dans des tumeurs telles que le cancer à petites cellules du poumon, alors que le gène ULIP correspondant est absent dans un tissu sain, ainsi que la modulation de l'expression des membres de la famille ULIP obtenus lors de la différenciation par le rétrovirus humain HTLV1 d'une lignée de médulloblastome, suggèrent l'implication des ULIP dans les tumeurs cancéreuses.

### EXEMPLE 12 :

### Production d'anticorps spécifiques de chacune des protéines ULIP humaines

Des peptides spécifiques de chaque membre de la famille ULIP ont été synthétisés synthétisé sur un appareil de synthèse peptidique multiple utilisant le F-moc (432A Peptide Synthesizer SYNERGY, Applied Biosystems). La pureté a été vérifiée par analyse de la séquence par HPLC et spectrométrie de masse.

Ces peptides sont :
Peptide spécifique de ULIP-1 G S A R G S P T R P N (11 acides aminés)
Peptide spécifique de ULIP-2 : S S A K T S P A K Q Q A (12 acides aminés)
Peptide spécifique de ULIP-3 : P S A K S S P S K H Q (11 acides aminés)
Peptide spécifique de ULIP-4 : P A R A S C P G K I S (11 acides aminés).

1 mg du peptide synthétique conjugué à de l'hémocyanine de patelle, dans de l'adjuvant complet de Freund, a été utilisé pour immuniser des lapins avec une dose « booster » de 0,5 mg de peptide lié dans de l'adjuvant complet de Freund après 4 semaines.

Les anticorps obtenus reconnaissent spécifiquement chaque protéine membre de la famille ULIP.

### EXEMPLE 13 :

### Production d'animaux transgéniques exprimant ULIP-4

Des drosophiles ont été transformées par l'ADNc d'ULIP-4 humain.

L'ADNc de ULIP-4, précédemment cloné dans pbluescript SK-phagemid, a été excisé par double digestion enzymatique Kpn1 et Xba1. Après électrophorèse sur gel d'agarose, le fragment d'ADNc a été purifié puis cloné dans pUAST, dérivant de pCaSpeR3, digéré par les enzymes de restriction Kpn1 et Xba1. Le plasmide 10-C résulte du clonage directionnel de l'AONc de ULIP-4 dans pUAST associé au gène rapporteur mini-white. Le plasmid 10-C a été injecté avec un plasmide helper p-delta-2-3 codant pour la transposase de l'élément P active dans la lignée germinale.

Les drosophiles transformées sont identifiées par leurs yeux rouges résultant de l'expression du gène mini-white. Ces lignées transformées par l'ADNc de ULIP-4 sous le contrôle de séquences régulatrices UASGAL4 permettent une expression ciblée de l'ADNc de ULIP-4.

Cette production de drosophiles transformées permet d'étudier spécifiquement le rôle de ULIP-4 dans différentes cellules et comprendre son implication dans les pathologies humaines.

### BIBLIOGRAPHIE

- Anderson et al., CRC Crit. Rev. Neurobiol., 1987. vol. 3, pp 245-99
- Antoine J.C. et al., Journal of the Neurological Sciences, 1993, vol. 117, pp 215-223
- Byk et al., Journal of Neuroscience, 1996, vol. 16(2), pp 688-701
- Chomczynksky et Sacchi, Anal. Biochem., 1987, 162 :156-159,
- Dalmau et al., Neurology, 1991, vol. 41, pp 1757-64
- Duchemin et al., Dev Neurosci, 1987, vol. 9, pp 61-67
- Graus et al., Neurology, 1985, vol. 35, pp 538-543
- Graus et al., Neurology, 1990, vol. 40, pp 219-22
- Greenlee et al., Ann. Neurol., 1983, vol, 14, pp 609-13
- Hamajima et al., Gene, 1996, vol. 180, pp. 157-163
- Hetzel et al., Mayo Clin. Proc., 1990, vol. 65, pp 1558-63
- Honnorat J. et al., Journal of Neurology, Neurosurgery and Psychiatry, 1996, vol. 61, pp 270-278
- Jaeckle et al., Ann. Neurol., 1985, vol. 18, pp 592-600
- Köhler et Milstein, Nature, 1975, vol. 256, pp 495-497
- Levy N., Mattei MG., 1995, Geneprobs II, a practical approch. BD Hames and SJ Higgins, Oxford University Press, pp 211-243
- Luque et al., Ann. Neurol., 1991, vol. 29, pp 241-51
- Minturn et al., J. Neurosci., 1995, vol. 15, pp. 6757-6766
- Nikali et al., Am. J. Hum. Genet., 1995, 56 :1088-1095
- Peltre G., Lapeyre J ; David B., Immunol. Lett., 1982, vol. 5 pp 127-131
- Sambrook et al., Molecular Cloning, a laboratory Manual, 1989, 9.47-9.62
- Varilo et al., Genome Res., 1996, 6 :870-875
- Wang L-H et al., J. Neurosci., 1996, vol. 16(9), pp 6197-6207

## Revendications

1. Polypeptide purifié comprenant la séquence d'acides aminés SEQ ID n° 8, ledit polypeptide étant désigné par « POP-66 ».

2. Acide nucléotidique isolé comprenant une séquence codant pour un polypeptide de séquence d'acides aminés SEQ ID n° 8.

3. Acide nucléique selon la revendication 2, comprenant une séquence SEQ ID n° 7.

4. Vecteur de clonage et/ou d'expression contenant une séquence d'acides nucléiques selon l'une des revendications 2 ou 3.

5. Cellule hôte transfectée par un vecteur selon la revendication 4.

6. Anticorps monoclonal dirigé contre un polypeptide POP-66 selon la revendication 1, ainsi que les fragments Fab ou F(ab')₂, ou les immunoconjugués dudit anticorps monoclonal.

7. Utilisation d'un polypeptide purifié POP-66 selon la revendication 1, pour détecter la présence d'anticorps anti-CV2 dans un échantillon biologique.

8. Utilisation d'un anticorps monoclonal ou ses fragments Fab ou F(ab')₂, ou immunoconjugués selon la revendication 6, pour la purification ou la détection d'une protéine POP-66 dans un échantillon biologique.

9. Méthode in vitro pour le diagnostic des syndromes neurologiques paranéoplasiques et/ou pour le diagnostic précoce de la formation des tumeurs cancéreuses, **caractérisée en ce que** l'on met en évidence dans un échantillon de sang prélevé chez un individu des auto-anticorps dirigés contre une protéine POP-66 par
- la mise en contact un échantillon de sang d'un individu avec un polypeptide purifié (POP-66) selon la revendication 1, éventuellement fixé sur un support dans des conditions permettant la formation de complexes immunologiques spécifiques entre ledit polypeptide et les auto-anticorps éventuellement présents dans l'échantillon de sang, et
- la détection des complexes immunologiques spécifiques éventuellement formés.

10. Kit pour le diagnostic des syndromes neurologiques paranéoplasiques et pour le diagnostic précoce de la formation des tumeurs à partir d'un prélèvement biologique comprenant :
- au moins un polypeptide purifié POP-66, selon la revendication 1, éventuellement fixé sur un support,
- des moyens de révélation de la formation de complexes antigène/anticorps spécifiques entre un auto-anticorps anti-POP-66 et ledit polypeptide purifié POP-66, et/ou des moyens de quantification de ces complexes.

11. Composition pharmaceutique, comprenant au moins un polypeptide purifié POP-66 selon la revendication 1, un acide nucléique codant pour ledit polypeptide, une séquence anti-sens capable de s'hybrider spécifiquement avec une séquence nucléotidique codant pour ledit polypeptide, ou un anticorps dirigé contre ledit polypeptide, associé à un véhicule pharmaceutiquement acceptable.

## Patentansprüche

1. Gereinigtes Polypeptid, umfassend die Aminosäuresequenz SEQ. ID. NR. 8, wobei das Polypeptid als "POP-66" bezeichnet wird.

2. Isolierte Nukleinsäure, umfassend eine Sequenz, die für ein Polypeptid der Aminosäuresequenz SEQ. ID. NR. 8 codiert.

3. Nukleinsäure nach Anspruch 2, umfassend eine Sequenz SEQ. ID. NR. 7.

4. Klonierungs- und/oder Expressionsvektor, der eine Sequenz von Nukleinsäuren nach einem der Ansprüche 2 oder 3 enthält.

5. Wirtszelle, die mit einem Vektor nach Anspruch 4 transfiziert ist.

6. Monoklonaler Antikörper, der gegen ein POP-66-Polypeptid nach Anspruch 1 gerichtet ist, sowie die Fab- oder F(ab')₂-Fragmente oder die Immunkonjugate des monoklonalen Antikörpers.

7. Verwendung eines gereinigten POP-66-Polypeptids nach Anspruch 1 zum Nachweis der Anwesenheit von Anti-CV2-Antikörpern in einer biologischen Probe.

8. Verwendung eines monoklonalen Antikörpers oder seiner Fab- oder F(ab')₂-Fragmente oder Immunkonjugate nach Anspruch 6 zur Reinigung oder zum Nachweis eines POP-66-Proteins in einer biologischen Probe.

9. In-vitro-Verfahren zur Diagnose paraneoplastischer, neurologischer Syndrome und/oder zur frühzeitigen Diagnose der Bildung kanzerogener Tumore, **dadurch gekennzeichnet, dass** man in einer von einem Individuum entnommenen Blutprobe Autoantikörper, die gegen ein POP-66-Protein gerichtet sind, nachweist durch
- In-Kontakt-Bringen einer Blutprobe eines Individuums mit einem gereinigten Polypeptid (POP-66) nach Anspruch 1, das gegebenenfalls an einem Träger fixiert ist, unter Bedingungen, die die Bildung spezifischer Immunkomplexe zwischen dem Polypeptid und den gegebenenfalls in der Blutprobe vorhandenen Autoantikörpern ermöglichen, und
- Nachweisen der gegebenenfalls gebildeten spezifischen Immunkomplexe.

10. Kit zur Diagnose paraneoplastischer, neurologischer Syndrome und zur frühzeitigen Diagnose der Bildung von Tumoren, ausgehend von einer biologischen Probe, umfassend:
- mindestens ein gereinigtes POP-66-Polypeptid nach Anspruch 1, das gegebenenfalls an einem Träger fixiert ist,
- Mittel zum Nachweis der Bildung spezifischer Antigen/Antikörper-Komplexe zwischen einem Anti-POP-66-Autoantikörper und dem gereinigten POP-66-Polypeptid und/oder Mittel zur Quantifizierung dieser Komplexe.

11. Pharmazeutische Zusammensetzung, umfassend mindestens ein gereinigtes POP-66-Polypeptid nach Anspruch 1, eine Nukleinsäure, die für das Polypeptid codiert, eine Antisense-Sequenz, die in der Lage ist, spezifisch mit einer für das Polypeptid codierenden Nukleotidsequenz zu hybridisieren, oder einen gegen das Polypeptid gerichteten Antikörper, in Verbindung mit einem pharmazeutisch annehmbaren Vehikel.

## Claims

1. Purified polypeptide comprising the aminated acid sequence SEQ ID No. 8, said polypeptide being designated as "POP-66".

2. Isolated nucleotide acid comprising a sequence encoding a polypeptide of the aminated acid sequence SEQ ID No.8.

3. Nucleic acid according to Claim 2 comprising a sequence SEQ ID No.7.

4. Cloning and/or expression vector containing a nucleic acid sequence according to one of Claims 2 or 3.

5. Host cell transfected by a vector according to Claim 4.

6. Monoclonal antibody directed against a POP-66 polypeptide according to Claim 1, as well as the Fab or F(ab')₂ fragments or immunoconjugates of said monoclonal antibody.

7. Use of a POP-66 purified polypeptide according to Claim 1 to detect the presence of anti-CV2 antibodies in a biological sample.

8. Use of a monoclonal antibody or its Fab or F(ab')₂ fragments or immunoconjugates according to Claim 6 for purification or detection of a POP-66 protein in a biological sample.

9. In vitro method for the diagnosis of neurological paraneoplastic syndromes and/or for the early diagnosis of the formation of cancerous tumours, **characterised in that** autoantibodies directed against a POP-66 protein are revealed in a blood sample taken from an individual by
• bringing a blood sample of an individual into contact with a purified polypeptide (POP-66) according to Claim 1. possibly fixed to a support in conditions allowing the formation of specific immunological complexes between said polypeptide and autoantibodies possibly present in the blood sample, and
• detecting specific immunological complexes possibly formed.

10. Kit for the diagnosis of neurological paraneoplastic syndromes and for the early diagnosis of the formation of tumours on the basis of a taken biological sample, comprising:
• at least one POP-66 purified polypeptide according to Claim 1, possibly fixed to a support,
• means for revealing the formation of specific antigen/antibody complexes between an anti-POP-66 autoantibody and said POP-66 purified polypeptide, and/or means for quantifying these complexes.

11. Pharmaceutical composition comprising at least one POP-66 purified polypeptide according to Claim 1, a nucleic acid encoding said polypeptide, an antisense sequence capable of specifically hybridising with a nucleotide sequence encoding said polypeptide, or an antibody directed against said polypeptide associated with a pharmaceutically acceptable vehicle.


### LISTE DE SEQUENCES

(1) INFORMATION GENERALE:
   (i) DEPOSANT:
      (A) NOM: Institut National de la Santé et de la Recherche Médicale
      (B) RUE: 101, rue de Tolbiac
      (C) VILLE: Paris
      (E) PAYS: FRANCE
      (F) CODE POSTAL: 75013
      (G) TELEPHONE: 0144236000
      (H) TELECOPIE: 0145856856
   (ii) TITRE DE L'INVENTION: Utilisation des Ulip dans les SNP et les cancers associés
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: Patent In Release #1.0, Version #1.25 (OEB)
(2) INFORMATION POUR LA SEQ ID NO: 1:
   (i) CARACTERISTIQUES DE LA SEQUENCE:
      (A) LONGUEUR: 1817 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: Mus musculus
   (xi) DESCRIPTION DE LA SEQUENCE: SEQ ID NO: 1:
   CTTCCTCCCG CCCCCCGGAG AGATGTCTTA TCAGGGGAAG AAAAATATTC CACCCATCAC     60
   GAGCGATCGT CTTCTGATCA AAGGTGGCAA GATTGTGAAT GATGACCAGT CCTTCTATGC    120
   AGACATATAC ATGGAAGATG GGTTGATCAA GCAAATAGGA GAAAACCTGA TTGTACCAGG    180
   AGGGGTGAAG ACCATCGAAG CCCACTCCAG AATGGTGATT CCCGGAGGAA TTGACGTGCA    240
   TACTCGCTTC CAGATGCCTG ACCAGGGAAT GACATCCGCT GATGACTTCT TCCAGGGAAC    300
   CAAGGCGGCC CTGGCCGGGG GAACCACCAT GATCATTGAC CATGTTGTTC CTGAGCCCGG 360
   GACGAGCCTA TTGGCTGCCT TTGATCAGTG GAGGGAGTGG GCTGACAGCA AGTCCTGCTG 420
   TGACTATTCG CTGCACGTGG ACATCACTGA GTGGCACAAG GGCATCCAGG AGGAGATGGA 480
   AGCTCTGGTG AAGGACCACG GGGTAAACTC CTTCCTCGTG TACATGGCTT TCAAAGATCG 540
   ATTCCAGCTG ACGGATTCCC AGATCTATGA AGTGCTGAGC GTGATCCGGG ATATCGGTGC 600
   CATAGCTCAA GTCCACGCAG AGAATGGTGA CATCATTGCT GAGGCACAGC AGAGGATCCT 660
   GGATCTGGGC ATCACGGGCC CCGAGGGACA CGTGTTGAGC CGGCCAGAGG AGGTCGAGGC 720
   TGAAGCTGTG AACCGGTCCA TCACTATTGC CAACCAGACC AACTGCCCTC TGTATGTCAC 780
   CAAAGTGATG CCCAAGAGTG CGGCTGAAGT CATCGCTCAG GCACGGAAGA AGGGAACTGT 840
   GGTGTATGGT GAGCCCATCA CGGCCAGCCT GGGGACTGAT GGCTCTCATT ACTGGAGCAA 900
   GAACTGGGCC AAGGCTGCGG CCTTTGTCAC CTCCCCACCC TTGAGCCCCG ACCCAACCAC 960
   TCCAGACTTT CTCAACTCGT TGCTGTCCTG TGGAGACCTC CAAGTCACTG GCAGTGCCCA 1020
   CTGCACCTTC AACACTGCCC AGAAGGCTGT GGGGAAGGAC AACTTCACCT TGATTCCCGA 1080
   GGGCACCAAC GGCACTGAGG AGCGGATGTC TGTCATTTGG GATAAAGCTG TGGTCACTGG 1140
   GAAGATGGAT GAGAATCAGT TTGTGGCTGT GACCAGCACC AACGCAGCCA AAGTCTTCAA 1200
   CCTTTACCCC CGGAAAGGTC GCATCTCGGT GGGATCTGAT GCTGACTTGG TCATCTGGGA 1260
   CCCTGACAGT GTGAAGACCA TCTCTGCCAA GACACACAAC AGTGCTCTTG AGTACAACAT 1320
   CTTTGAAGGC ATGGAGTGTC GCGGCTCCCC ACTGGTGGTC ATCAGCCAGG GCAAGATTGT 1380
   CCTGGAGGAC GGCACACTTC ATGTCACTGA AGGCTCAGGA CGCTACATTC CCCGGAAGCC 1440
   CTTCCCTGAC TTTGTGTACA AACGCATCAA AGCAAGGAGC AGGCTGGCTG AGCTGAGAGG 1500
   GGTCCCTCGT GGCCTGTATG ACGGACCGGT ATGCGAGGTG TCTGTGACGC CCAAGACGGT 1560
   GACTCCAGCC TCATCAGCTA AGACATCCCC TGCCAAGCAG CAGGCACCAC CTGTTCGGAA 1620
   CCTGCACCAG TCTGGATTCA GCTTGTCTGG TGCTCAGATT GACGACAACA TTCCCCGCCG 1680
   CACCACCCAG CGCATCGTGG CACCCCCTGG TGGCCGTGCC AACATCACCA GCCTGGGCTA 1740
   AAGCCCCTAG GCCTGCAGGC CACTTGGGGA TGGGGGATGG GACACCTGAG GACATTCTGA 1800
   GACTTCCTTT CTTCCAT 1817
(2) INFORMATION POUR LA SEQ ID NO: 2:
   (i) CARACTERISTIQUES DE LA SEQUENCE:
      (A) LONGUEUR: 572 acides aminés
      (B) TYPE: acide aminé
      (C) NOMBRE DE BRINS: simple
      (D) CONFIGURATION: linéaire
   (ii) TYPE DE MOLECULE: peptide
   (vi) ORIGINE:
      (A) ORGANISME: Mus musculus
   (xi) DESCRIPTION DE LA SEQUENCE: SEQ ID NO: 2:
   Met Ser Tyr Gln Gly Lys Lys Asn Ile Pro Pro Ile Thr Ser Asp Arg
   1               5                   10                  15
   Leu Leu Ile Lys Gly Gly Lys Ile Val Asn Asp Asp Gln Ser Phe Tyr
               20                  25                  30
   Ala Asp Ile Tyr Met Glu Asp Gly Leu Ile Lys Gln Ile Gly Glu Asn
           35                  40                  45
   Leu Ile Val Pro Gly Gly Val Lys Thr Ile Glu Ala His Ser Arg Met
       50                  55                  60
   Val Ile Pro Gly Gly Ile Asp Val His Thr Arg Phe Gln Met Pro Asp
   65                  70                  75                  80
   Gln Gly Met Thr Ser Ala Asp Asp Phe Phe Gln Gly Thr Lys Ala Ala
                   85                  90                  95
   Leu Ala Gly Gly Thr Thr Met Ile Ile Asp His Val Val Pro Glu Pro
               100                 105                 110
   Gly Thr Ser Leu Leu Ala Ala Phe Asp Gln Trp Arg Glu Trp Ala Asp
           115                 120                 125
   Ser Lys Ser Cys Cys Asp Tyr Ser Leu His Val Asp Ile Thr Glu Trp
       130                 135                 140
   His Lys Gly Ile Gln Glu Glu Met Glu Ala Leu Val Lys Asp His Gly
   145                 150                 155                 160
   Val Asn Ser Phe Leu Val Tyr Met Ala Phe Lys Asp Arg Phe Gln Leu
                   165                 170                 175
   Thr Asp Ser Gln Ile Tyr Glu Val Leu Ser Val Ile Arg Asp Ile Gly
               180                 185                 190
   Ala Ile Ala Gln Val His Ala Glu Asn Gly Asp Ile Ile Ala Glu Ala
           195                 200                 205
   Gln Gln Arg Ile Leu Asp Leu Gly Ile Thr Gly Pro Glu Gly His Val
       210                 215                 220
   Leu Ser Arg Pro Glu Glu Val Glu Ala Glu Ala Val Asn Arg Ser Ile
   225                 230                 235                 240
   Thr Ile Ala Asn Gln Thr Asn Cys Pro Leu Tyr Val Thr Lys Val Met
                   245                 250                 255
   Pro Lys Ser Ala Ala Glu Val Ile Ala Gln Ala Arg Lys Lys Gly Thr
               260                 265                 270
   Val Val Tyr Gly Glu Pro Ile Thr Ala Ser Leu Gly Thr Asp Gly Ser
           275                 280                 285
   His Tyr Trp Ser Lys Asn Trp Ala Lys Ala Ala Ala Phe Val Thr Ser
       290                 295                 300
   Pro Pro Leu Ser Pro Asp Pro Thr Thr Pro Asp Phe Leu Asn Ser Leu
   305                 310                 315                 320
   Leu Ser Cys Gly Asp Leu Gln Val Thr Gly Ser Ala His Cys Thr Phe
                   325                 330                 335
   Asn Thr Ala Gln Lys Ala Val Gly Lys Asp Asn Phe Thr Leu Ile Pro
               340                 345                 350
   Glu Gly Thr Asn Gly Thr Glu Glu Arg Met Ser Val Ile Trp Asp Lys
           355                 360                 365
   Ala Val Val Thr Gly Lys Met Asp Glu Asn Gln Phe Val Ala Val Thr
       370                 375                 380
   Ser Thr Asn Ala Ala Lys Val Phe Asn Leu Tyr Pro Arg Lys Gly Arg
   385                 390                 395                 400
   Ile Ser Val Gly Ser Asp Ala Asp Leu Val Ile Trp Asp Pro Asp Ser
                   405                 410                 415
   Val Lys Thr Ile Ser Ala Lys Thr His Asn Ser Ala Leu Glu Tyr Asn
               420                 425                 430
   Ile Phe Glu Gly Met Glu Cys Arg Gly Ser Pro Leu Val Val Ile Ser
           435                 440                 445
   Gln Gly Lys Ile Val Leu Glu Asp Gly Thr Leu His Val Thr Glu Gly
       450                 455                 460
   Ser Gly Arg Tyr Ile Pro Arg Lys Pro Phe Pro Asp Phe Val Tyr Lys
   465                 470                 475                 480
   Arg Ile Lys Ala Arg Ser Arg Leu Ala Glu Leu Arg Gly Val Pro Arg
                       485                 490                 495
   Gly Leu Tyr Asp Gly Pro Val Cys Glu Val Ser Val Thr Pro Lys Thr
                   500                 505                 510
   Val Thr Pro Ala Ser Ser Ala Lys Thr Ser Pro Ala Lys Gln Gln Ala
               515                 520                 525
   Pro Pro Val Arg Asn Leu His Gln Ser Gly Phe Ser Leu Ser Gly Ala
       530                 535                 540
   Gln Ile Asp Asp Asn Ile Pro Arg Arg Thr Thr Gln Arg Ile Val Ala
   545                 550                 555                 560
   Pro Pro Gly Gly Arg Ala Asn Ile Thr Ser Leu Gly
                   565                 570
(2) INFORMATION POUR LA SEQ ID NO: 3:
   (i) CARACTERISTIQUES DE LA SEQUENCE:
      (A) LONGUEUR: 2297 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: Mus musculus
   (xi) DESCRIPTION DE LA SEQUENCE: SEQ ID NO: 3:
   GCTGTCTGTC TTCAGCGCCC TCCTCTCGCC CTGCCTCTCC CTCCTCCTCC CGCCCTCCTT     60
   GCCAAGCCGG GCGGTGCAGG CAGCCGGAGC AGCGGCGGCG GGCCGAGCAG CGGGGAGTGG    120
   GCAGCGGTGG GAGCCGAGCT TCTGTCCTTT CTTTCATCCC TCCCTGGCCT TTGTCGCCGC    180
   TCTCACGAGT AGCGCCGCCG GGAGAGACCC GGGTAGAGCG CCAGGCAGAC GTTAGTTCCA    240
   GCGGCCGGGC GGAGGGCTCC AGAGGGGCCA TGTCTCATCA GGGGAAGAAG AGCATCCCGC    300
   ACATCACCAG TGACCGGCTC CTCATCAGAG GTGGACGCAT CATCAATGAT GACCAGTCCT    360
   TCTACGCCGA TGTCTACCTA GAAGATGGAC TCATAAAACA AATAGGAGAG AACCTGATTG    420
   TTCCTGGTGG AGTGAAGACC ATCGAGGCGA ATGGCCGAAT GGTCATTCCC GGTGGCATTG    480
   ATGTCAACAC TTACCTGCAG AAGCCCTCCC AGGGCATGAC CTCGGCTGAT GACTTCTTCC    540
   AGGGCACTAA AGCAGCGCTG GCAGGTGGAA CCACGATGAT CATTGACCAC GTTGTTCCTG    600
   AACCTGGGTC CAGCTTGTTG ACTTCCTTTG AGAAATGGCA CGAAGCAGCA GACACCAAAT 660
   CCTGCTGTGA CTATTCCCTC CACGTGGACA TCACAAGCTG GTATGATGGT GTTCGGGAAG 720
   AGCTGGAGGT GCTGGTGCAG GACAAAGGTG TCAACTCCTT CCAAGTCTAC ATGGCGTATA 780
   AGGACCTGTA CCAGATGTCT GACAGCCAGC TGTATGAAGC CTTCACCTTC CTTAAGGGTT 840
   TGGGAGCTGT GATCTTAGTC CATGCAGAAA ATGGAGATTT GATAGCTCAG GAACAAAAAC 900
   GGATCCTGGA GATGGGCATC ACGGGTCCCG AGGGTCATGC TCTGAGCAGA CCCGAGGAGC 960
   TGGAGGCCGA GGCTGTGTTC CGGGCTATTG CCATTGCAGG CCGGATCAAT TGCCCTGTGT 1020
   ACATCACCAA GGTCATGAGC AAGAGTGCAG CGGACATCAT CGCACTGGCC AGGAAGAAAG 1080
   GCCCTCTTGT CTTCGGTGAG CCCATAGCCG CCAGCCTGGG AACCGATGGC ACCCACTACT 1140
   GGAGCAAGAA CTGGGCCAAG GCAGCTGCAT TTGTGACTTC CCCTCCCCTG AGCCCAGACC 1200
   CCACCACTCC TGACTACTTG ACCTCCTTGC TGGCCTGTGG AGACTTGCAG GTCACAGGTA 1260
   GTGGCCACTG TCCCTACAGT ATTGCTCAGA AGGCTGTGGG CAAGGACAAC TTCACTCTGA 1320
   TCCCTGAGGG TGTCAATGGT ATAGAAGAGC GGATGACCGT TGTCTGGGAC AAGGCAGTGG 1380
   CTACTGGCAA GATGGATGAG AACCAGTTTG TAGCCGTCAC CAGCACCAAC GCAGCCAAGA 1440
   TCTTCAACCT GTACCCGAGG AAAGGTCGGA TCGCTGTGGG CTCCGATGCT GACGTAGTCA 1500
   TCTGGGACCC AGATAAGATG AAGACCATAA CAGCCAAAAG CCATAAATCA ACTGTGGAGT 1560
   ACAACATCTT TGAGGGCATG GAGTGCCACG GCTCCCCCCT GGTGGTCATC AGTCAGGGCA 1620
   AGATTGTCTT TGAGGATGGA AACATCAGTG TCAGCAAGGG CATGGGCCGC TTCATCCCTC 1680
   GGAAGCCATT CCCAGAGCAT CTCTACCAGC GTGTCAGGAT CAGAAGCAAG GTTTTCGGGT 1740
   TGCATAGTGT TTCCAGGGGC ATGTACGATG GGCCTGTGTA CGAGGTGCCA GCTACACCCA 1800
   AACATGCTGC TCCTGCTCCT TCTGCCGAAT CCTCGCCTTC TAAACACCAA CCCCCACCCA 1860
   TCCGGAACCT CCACCAGTCC AACTTCAGCT TATCAGGTGC CCAGATAGAT GACAACAATC 1920
   CAAGGCGTAC AGGCCACCGC ATTGTGGCGC CCCCTGGTGG CCGCTCCAAC ATCACCAGCC 1980
   TCGGTTGACC TCAGATGAGC CAGATATGCA AGAGTGAAGG ATTATGGGAA AACGTCCATT 2040
   CCTTTTCCGT GTTTTTGAAG CCCACAGTTT TAGTTGGTAC TGACGGAGGG GAGATTGAGC 2100
   GATGCTCTTT CCTTCTCTGT TTAGGAAGAA GTGGTACTAG TGTGGTGTGT TTGCCTGGAA 2160
   GTCCCTCGCC CACAGTGTGT GTTCACACCG ACTCCACCTC AGAGCATGGT GCCGTCCGTT 2220
   TTCCCTTCCT AGTGACCCCA GGTTTAGCAT CGTCCTATAC TGTTCCCTCC ACTCCTCCAT 2280
   GACCCTCTGA GTGATGG 2297
(2) INFORMATION POUR LA SEQ ID NO: 4:
   (i) CARACTERISTIQUES DE LA SEQUENCE:
      (A) LONGUEUR: 572 acides aminés
      (B) TYPE: acide aminé
      (C) NOMBRE DE BRINS: simple
      (D) CONFIGURATION: linéaire
   (ii) TYPE DE MOLECULE: peptide
   (vi) ORIGINE:
      (A) ORGANISME: Mus musculus
   (xi) DESCRIPTION DE LA SEQUENCE: SEQ ID NO: 4:
   Met Ser His Gln Gly Lys Lys Ser Ile Pro His Ile Thr Ser Asp Arg
   1               5                   10                  15
   Leu Leu Ile Arg Gly Gly Arg Ile Ile Asn Asp Asp Gln Ser Phe Tyr
               20                  25                  30
   Ala Asp Val Tyr Leu Glu Asp Gly Leu Ile Lys Gln Ile Gly Glu Asn
           35                  40                  45
   Leu Ile Val Pro Gly Gly Val Lys Thr Ile Glu Ala Asn Gly Arg Met
       50                  55                  60
   Val Ile Pro Gly Gly Ile Asp Val Asn Thr Tyr Leu Gln Lys Pro Ser
   65                  70                  75                  80
   Gln Gly Met Thr Ser Ala Asp Asp Phe Phe Gln Gly Thr Lys Ala Ala
                   85                  90                  95
   Leu Ala Gly Gly Thr Thr Met Ile Ile Asp His Val Val Pro Glu Pro
               100                 105                 110
   Gly Ser Ser Leu Leu Thr Ser Phe Glu Lys Trp His Glu Ala Ala Asp
           115                 120                 125
   Thr Lys Ser Cys Cys Asp Tyr Ser Leu His Val Asp Ile Thr Ser Trp
       130                 135                 140
   Tyr Asp Gly Val Arg Glu Glu Leu Glu Val Leu Val Gln Asp Lys Gly
   145                 150                 155                 160
   Val Asn Ser Phe Gln Val Tyr Met Ala Tyr Lys Asp Leu Tyr Gln Met
                   165                 170                 175
   Ser Asp Ser Gln Leu Tyr Glu Ala Phe Thr Phe Leu Lys Gly Leu Gly
               180                 185                 190
   Ala Val Ile Leu Val His Ala Glu Asn Gly Asp Leu Ile Ala Gln Glu
           195                 200                 205
   Gln Lys Arg Ile Leu Glu Met Gly Ile Thr Gly Pro Glu Gly His Ala
       210                 215                 220
   Leu Ser Arg Pro Glu Glu Leu Glu Ala Glu Ala Val Phe Arg Ala Ile
   225                 230                 235                 240
   Ala Ile Ala Gly Arg Ile Asn Cys Pro Val Tyr Ile Thr Lys Val Met
                   245                 250                 255
   Ser Lys Ser Ala Ala Asp Ile Ile Ala Leu Ala Arg Lys Lys Gly Pro
               260                 265                 270
   Leu Val Phe Gly Glu Pro Ile Ala Ala Ser Leu Gly Thr Asp Gly Thr
           275                 280                 285
   His Tyr Trp Ser Lys Asn Trp Ala Lys Ala Ala Ala Phe Val Thr Ser
       290                 295                 300
   Pro Pro Leu Ser Pro Asp Pro Thr Thr Pro Asp Tyr Leu Thr Ser Leu
   305                 310                 315                 320
   Leu Ala Cys Gly Asp Leu Gln Val Thr Gly Ser Gly His Cys Pro Tyr
                   325                 330                 335
   Ser Ile Ala Gln Lys Ala Val Gly Lys Asp Asn Phe Thr Leu Ile Pro
               340                 345                 350
   Glu Gly Val Asn Gly Ile Glu Glu Arg Met Thr Val Val Trp Asp Lys
           355                 360                 365
   Ala Val Ala Thr Gly Lys Met Asp Glu Asn Gln Phe Val Ala Val Thr
       370                 375                 380
   Ser Thr Asn Ala Ala Lys Ile Phe Asn Leu Tyr Pro Arg Lys Gly Arg
   385                 390                 395                 400
   Ile Ala Val Gly Ser Asp Ala Asp Val Val Ile Trp Asp Pro Asp Lys
                   405                 410                 415
   Met Lys Thr Ile Thr Ala Lys Ser His Lys Ser Thr Val Glu Tyr Asn
               420                 425                 430
   Ile Phe Glu Gly Met Glu Cys His Gly Ser Pro Leu Val Val Ile Ser
           435                 440                 445
   Gln Gly Lys Ile Val Phe Glu Asp Gly Asn Ile Ser Val Ser Lys Gly
               450                 455                 460
   Met Gly Arg Phe Ile Pro Arg Lys Pro Phe Pro Glu His Leu Tyr Gln
   465                 470                 475                 480
   Arg Val Arg Ile Arg Ser Lys Val Phe Gly Leu His Ser Val Ser Arg
                   485                 490                 495
   Gly Met Tyr Asp Gly Pro Val Tyr Glu Val Pro Ala Thr Pro Lys His
               500                 505                 510
   Ala Ala Pro Ala Pro Ser Ala Glu Ser Ser Pro Ser Lys His Gln Pro
           515                 520                 525
   Pro Pro Ile Arg Asn Leu His Gln Ser Asn Phe Ser Leu Ser Gly Ala
       530                 535                 540
   Gln Ile Asp Asp Asn Asn Pro Arg Arg Thr Gly His Arg Ile Val Ala
   545                 550                 555                 560
   Pro Pro Gly Gly Arg Ser Asn Ile Thr Ser Leu Gly
                   565                 570
(2) INFORMATION POUR LA SEQ ID NO: 5:
   (i) CARACTERISTIQUES DE LA SEQUENCE:
      (A) LONGUEUR: 1920 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: Mus musculus
   (xi) DESCRIPTION DE LA SEQUENCE: SEQ ID NO: 5:
   GCTGACTAAT ATGCTTAAAT TCAGCGGGTC GCCACGTCTG GTCGGTACGT CCACGCCCGC    60
   GCAGCCCCTA CCGAGGACAC TCAGCCCGCC CGTGTATCAG GATGTCCTTC CAAGGCAAGA   120
   AGAGCATTCC CCGGATAACG AGCGACCGCC TTCTCATCAA AGGTGGGAAG ATTGTGAACG   180
   ATGACCAGTC CTTTCATGCT GATCTGTATG TGGAAGACGG TCTGATTAAA CAAATTGGAG   240
   AAAATCTCAT CGTCCCTGGG GGCATCAAAA CCATCGATGC TCATGGCCTG ATGGTGCTGC   300
   CTGGGGGAGT TGACGTTCAC ACCCGGCTGC AGATGCCTGT GATGGGCATG ACCCCAGCTG   360
   ATGATTTCTG TCAGGGCACC AAGGCGGCTC TAGCAGGCGG GACCACCATG ATATTGGACC   420
   ATGTGTTTCC TGACGCTGGT GTGAGCCTGC TGGCAGCCTA TGAGCAGTGG CGGGACGGAG    480
   CAGACAGCGC GGCCTGCTGT GACTACTCCT TACATGTGGA CATTCCTCGC TGGCACGAGA    540
   GCACCAAAGA AGAGCTGGAG GCCCTAGTCA GGGACAAAGG TGTGAACTCC TTCCTGGTCT    600
   TCATGGCATA CAAGGACAGG TGCCAGTGTA CTGACGGTCA GATATATGAA ATCTTCAGCC    660
   TCATCCGGGA CCTGGGAGCT GTGGCCCAGG TGCACGCAGA AAATGGGGAC ATCGTGGAGG    720
   AGGAACAGAA GCGCCTGCTG GAGCAAGGCA TCACTGGTCC TGAGGGCCAT GTGCTCAGCC    780
   ACCCAGAAGA GGTAGAGGCC GAGGCTGTGT ACAGAGCAGT CACCATTGCC AAGCAGGCCA    840
   ACTGCCCACT ATACGTCACC AAGGTGATGA GCAAGGGTGC AGCTGACATG GTTGCCCAAG    900
   CCAAGCGCAG GGGGGTGGTC GTCTTTGGGG AACCTATCAC TGCCAGCCTG GGCACTGATG    960
   GCTCACACTA CTGGAGCAAG AACTGGGCCA AGGCTGCAGC CTTTGTCACT TCACCCCCTA   1020
   TCAACCCGGA CCCTACTACT GCAGACCACC TCACCTCTCT GCTGTCCAGT GGGGACCTCC   1080
   AGGTGACAGG CAGTGCCCAC TGCACCTTCA CTACTGCCCA GAAGGCTGTT GGCAAAGACA   1140
   ACTTCACACT GATCCCCGAG GTAGTCAACG GTATAGAAGA GCGCATGTCT GTGGTCTGGG   1200
   AGAAATGTGT GGCTTCAGGG AAAATGGACG AGAATGAGTT CGTTGCCGTG ACCAGCACAA   1260
   ATGCTGCCAA AATCTTCAAT TTTTACCCCA GGAAGGGGCG TGTGGCCGTG GGCTCTGATG   1320
   CTGACCTGGT CATCTGGAAC CCCAGGGCCA CGAAAGTCAT CTCTGCCAAG AGCCATAACC   1380
   TGAATGTAGA GTACAACATC TTTGAAGGAG TGGAGTGCCG AGGAGTGCCC ACGGTGGTCA   1440
   TAAGTCAGGG CAGAGTGGTG CTGGAGGACG GAAACCTGCT TGTCACTCCA GGGGCTGGCC   1500
   GCTTCATTCC CCGGAAGACG TTCCCGGACT TTGTCTATAA GAGGATAAAG GCTCGCAACA   1560
   GGCTAGCAGA GATCCACGGT GTGCCTCGAG GCCTGTACGA CGGGCCTGTG CATGAAGTGA   1620
   TGTTACCTGC CAAGCCAGGA AGTGGCACAC AGGCCCGTGC ATCCTGTTCA GGCAAGATCT   1680
   CAGTGCCACC CGTGCGCAAC CTGCACCAGT CGGGGTTCAG CCTATCTGGC TCTCAGGCTG   1740
   ACGATCACAT TGCCAGACGT ACGGCTCAGA AGATCATGGC ACCCCCCGGA GGACGCTCCA   1800
   ACATCACGTC TCTTTCCTAG ACTTGGGGTC TTGGCAAGCT GGTGCTGTCC CCACTGGCAG   1860
   GGTGTGGGGA CGACTCACGT CAGTTAGCTC CTTCCTTTGT AGATTGTTAT TGTGAAAGGC   1920
(2) INFORMATION POUR LA SEQ ID NO 6:
   (i) CARACTERISTIQUES DE LA SEQUENCE:
      (A) LONGUEUR: 572 acides aminés
      (B) TYPE: acide aminé
      (C) NOMBRE DE BRINS: simple
      (D) CONFIGURATION: linéaire
   (ii) TYPE DE MOLECULE: peptide
   (vi) ORIGINE:
      (A) ORGANISME: Mus musculus
   (xi) DESCRIPTION DE LA SEQUENCE: SEQ ID NO 6:
   Met Ser Phe Gln Gly Lys Lys Ser Ile Pro Arg Ile Thr Ser Asp Arg
   1               5                   10                  15
   Leu Leu Ile Lys Gly Gly Lys Ile Val Asn Asp Asp Gln Ser Phe His
               20                  25                  30
   Ala Asp Leu Tyr Val Glu Asp Gly Leu Ile Lys Gln Ile Gly Glu Asn
           35                  40                  45
   Leu Ile Val Pro Gly Gly Ile Lys Thr Ile Asp Ala His Gly Leu Met
       50                  55                  60
   Val Leu Pro Gly Gly Val Asp Val His Thr Arg Leu Gln Met Pro Val
   65                  70                  75                  80
   Met Gly Met Thr Pro Ala Asp Asp Phe Cys Gln Gly Thr Lys Ala Ala
                   85                  90                  95
   Leu Ala Gly Gly Thr Thr Met Ile Leu Asp His Val Phe Pro Asp Ala
               100                 105                 110
   Gly Val Ser Leu Leu Ala Ala Tyr Glu Gln Trp Arg Asp Gly Ala Asp
           115                 120                 125
   Ser Ala Ala Cys Cys Asp Tyr Ser Leu His Val Asp Ile Pro Arg Trp
       130                 135                 140
   His Glu Ser Thr Lys Glu Glu Leu Glu Ala Leu Val Arg Asp Lys Gly
   145                 150                 155                 160
   Val Asn Ser Phe Leu Val Phe Met Ala Tyr Lys Asp Arg Cys Gln Cys
                   165                 170                 175
   Thr Asp Gly Gln Ile Tyr Glu Ile Phe Ser Leu Ile Arg Asp Leu Gly
               180                 185                 190
   Ala Val Ala Gln Val His Ala Glu Asn Gly Asp Ile Val Glu Glu Glu
           195                 200                 205
   Gln Lys Arg Leu Leu Glu Gln Gly Ile Thr Gly Pro Glu Gly His Val
       210                 215                 220
   Leu Ser His Pro Glu Glu Val Glu Ala Glu Ala Val Tyr Arg Ala Val
   225                 230                 235                 240
   Thr Ile Ala Lys Gln Ala Asn Cys Pro Leu Tyr Val Thr Lys Val Met
                   245                 250                 255
   Ser Lys Gly Ala Ala Asp Met Val Ala Gln Ala Lys Arg Arg Gly Val
               260                 265                 270
   Val Val Phe Gly Glu Pro Ile Thr Ala Ser Leu Gly Thr Asp Gly Ser
           275                 280                 285
   His Tyr Trp Ser Lys Asn Trp Ala Lys Ala Ala Ala Phe Val Thr Ser
       290                 295                 300
   Pro Pro Ile Asn Pro Asp Pro Thr Thr Ala Asp His Leu Thr Ser Leu
   305                 310                 315                 320
   Leu Ser Ser Gly Asp Leu Gln Val Thr Gly Ser Ala His Cys Thr Phe
                   325                 330                 335
   Thr Thr Ala Gln Lys Ala Val Gly Lys Asp Asn Phe Thr Leu Ile Pro
               340                 345                 350
   Glu Val Val Asn Gly Ile Glu Glu Arg Met Ser Val Val Trp Glu Lys
           355                 360                 365
   Cys Val Ala Ser Gly Lys Met Asp Glu Asn Glu Phe Val Ala Val Thr
       370                 375                 380
   Ser Thr Asn Ala Ala Lys Ile Phe Asn Phe Tyr Pro Arg Lys Gly Arg
   385                 390                 395                 400
   Val Ala Val Gly Ser Asp Ala Asp Leu Val Ile Trp Asn Pro Arg Ala
                   405                 410                 415
   Thr Lys Val Ile Ser Ala Lys Ser His Asn Leu Asn Val Glu Tyr Asn
               420                 425                 430
   Ile Phe Glu Gly Val Glu Cys Arg Gly Val Pro Thr Val Val Ile Ser
           435                 440                 445
   Gln Gly Arg Val Val Leu Glu Asp Gly Asn Leu Leu Val Thr Pro Gly
       450                 455                 460
   Ala Gly Arg Phe Ile Pro Arg Lys Thr Phe Pro Asp Phe Val Tyr Lys
   465                 470                 475                 480
   Arg Ile Lys Ala Arg Asn Arg Leu Ala Glu Ile His Gly Val Pro Arg
                   485                 490                 495
   Gly Leu Tyr Asp Gly Pro Val His Glu Val Met Leu Pro Ala Lys Pro
               500                 505                 510
   Gly Ser Gly Thr Gln Ala Arg Ala Ser Cys Ser Gly Lys Ile Ser Val
           515                 520                 525
   Pro Pro Val Arg Asn Leu His Gln Ser Gly Phe Ser Leu Ser Gly Ser
       530                 535                 540
   Gln Ala Asp Asp His Ile Ala Arg Arg Thr Ala Gln Lys Ile Met Ala
   545                 550                 555                 560
   Pro Pro Gly Gly Arg Ser Asn Ile Thr Ser Leu Ser
                   565                 570
(2) INFORMATION POUR LA SEQ ID NO 7 :
   (i) CARACTERISTIQUES DE LA SEQUENCE:
      (A) LONGUEUR: 1690 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: Homo sapiens
   (xi) DESCRIPTION DE LA SEQUENCE: SEQ ID NO 7:
   GCCGCCCCTA CCAGAGACCC CCAGGAGCAG GATGTCCTTC CAGGGCAAGA AAAGCATCCC    60
   CCGGATCACG AGTGACCGCC TTCTGATCAG AGGTGGGAGG ATCGTGAATG ACGACCAGTC   120
   CTTTTACGCT GATGTGCACG TGGAAGATGG CTTGATAAAA CAAATCGGAG AAAACCTCAT   180
   CGTCCCTGGG GGCATCAAGA CCATTGACGC CCACGGCCTG ATGGTCCTTC CTGGTGGCGT   240
   TGACGTCCAC ACAAGGCTGC AGATGCCTGT CCTGGGCATG ACACCGGCTG ACGACTTCTG   300
   TCAGGGCACC AAGGCAGCGC TAGCAGGAGG AACCACCATG ATCTTGGACC ACGTCTTCCC   360
   CGACACGGGT GTGAGCCTGC TGGCGGCCTA CGAGCAGTGG CGGGAGCGGG CGGACAGCGC   420
   GGCCTGCTGC GACTACTCCC TGCACGTGGA CATCACCCGA TGGCATGAGA GCATCAAGGA   480
   GGAGCTGGAG GCCCTGGTCA AGGAGAAGGG TGTGAACTCC TTCCTGGTCT TCATGGCATA   540
   CAAGGACCGG TGCCAGTGCA GCGACAGCCA GATGTACGAG ATCTTCAGCA TCATCCGGGA   600
   CCTGGGGGCC TTGGCCCAGG TGCACGCTGA GAACGGGGAC ATCGTGGAGG AGGAGCAGAA   660
   GCGGTTGCTG GAGCTCGGCA TCACTGGCCC CGAGGGCCAC GTGCTCAGCC ACCCCGAGGA    720
   GGTGGAGGCT GAGGCGGTGT ACCGAGCTGT CACCATCGCC AAGCAGGCAA ACTGCCCGCT    780
   GTACGTCACC AAGGTGATGA GCAAGGGGGC GGCCGACGCC ATCGCTCAGG CCAAGCGCAG    840
   AGGGGTGGTC GTGTTTGGGG AGCCCATCAC CGCCAGCCTG GGCACCGACG GTTCACACTA    900
   CTGGAGCAAG AACTGGGCCA AGGCTGCAGC CTTCGTCACA TCACCCCCTG TCAACCCAGA    960
   CCCCACCACG GCAGACCACC TCACCTGCTT GCTGTCCAGC GGGGACCTCC AGGTGACAGG   1020
   CAGCGCCCAC TGCACCTTCA CCACTGCCCA GAAGGCTGTG GGCAAGGACA ACTTCGCGCT   1080
   GATCCCCGAG GGCACCAACG GCATTGAGGA GCGCATGTCG ATGGTCTGGG AGAAATGTGT   1140
   GGCCTCTGGG AAGATGGACG AGAATGAGTT CGTCGCGGTG ACCAGTACAA ATGCTGCCAA   1200
   AATCTTCAAT TTTTACCCAA GGAAGGGGCG AGTGGCTGTG GGCTCTGACG CTGACCTGGT   1260
   CATATGGAAC CCCAAGGCCA CCAAGATCAT CTCTGCCAAG ACCCACAATC TGAACGTGGA   1320
   GTACAACATC TTCGAGGGAG TGGAGTGCCG GGGAGCGCCT GCCGTGGTCA TAAGTCAGGG   1380
   CCGAGTGGCG CTGGAGGACG GGAAGATGTT TGTCACCCCG GGGGCGGGCC GCTTCGTCCC   1440
   TCGGAAAACA TTCCCGGACT TTGTCTACAA GAGGATCAAA GCTCGCAACA GGCTGGCGGA   1500
   GATCCACGGT GTGCCCCGTG GGCTGTATGA CGGGCCCGTC CACGAGGTGA TGGTGCCTGC   1560
   CAAGCCAGGG AGTGGCGCTC CGGCCCGCGC GTCCTGCCCA GGCAAGATCT CCGTGCCTCC   1620
   TGTGCGCAAC CTACATCAGT CGGGGTTCAG CCTATCTGGG TCTCAGGCTG ATGACCACAT   1680
   CGCCCGACGC                                                          1690
(2) INFORMATION POUR LA SEQ ID NO 8 :
   (i) CARACTERISTIQUES DE LA SEQUENCE:
      (A) LONGUEUR: 572 acides aminés
      (B) TYPE: acide aminé
      (C) NOMBRE DE BRINS: simple
      (D) CONFIGURATION: linéaire
   (ii) TYPE DE MOLECULE: peptide
   (vi) ORIGINE:
      (A) ORGANISME: Homo sapiens
   (xi) DESCRIPTION DE LA SEQUENCE: SEQ ID NO 8 :
   Met Ser Phe Gln Gly Lys Lys Ser Ile Pro Arg Ile Thr Ser Asp Arg
   1               5                   10                  15
   Leu Leu Ile Arg Gly Gly Arg Ile Val Asn Asp Asp Gln Ser Phe Tyr
               20                  25                  30
   Ala Asp Val His Val Glu Asp Gly Leu Ile Lys Gln Ile Gly Glu Asn
           35                  40                  45
   Leu Ile Val Pro Gly Gly Ile His Thr Ile Asp Ala His Gly Leu Met
       50                  55                  60
   Val Leu Pro Gly Gly Val Asp Val His Thr Arg Leu Gln Met Pro Val
   65                  70                  75                  80
   Leu Gly Met Thr Pro Ala Asp Asp Phe Cys Gln Gly Thr Lys Ala Ala
                   85                  90                  95
   Leu Ala Gly Gly Thr Thr Met Ile Leu Asp His Val Phe Pro Asp Thr
               100                 105                 110
   Gly Val Ser Leu Leu Ala Ala Tyr Glu Gln Trp Arg Glu Arg Ala Asp
           115                 120                 125
   Ser Ala Ala Cys Cys Asp Tyr Ser Leu His Val Asp Ile Thr Arg Trp
       130                 135                 140
   His Glu Ser Ile Lys Glu Glu Leu Glu Ala Leu Val Lys Glu Lys Gly
   145                 150                 155                 160
   Val Asn Ser Phe Leu Val Phe Met Ala Tyr Lys Asp Arg Cys Gln Cys
                   165                 170                 175
   Ser Asp Ser Gln Met Tyr Glu Ile Phe Ser Ile Ile Arg Asp Leu Gly
               180                 185                 190
   Ala Leu Ala Gln Val His Ala Glu Asn Gly Asp Ile Val Glu Glu Glu
           195                 200                 205
   Gln Lys Arg Leu Leu Glu Leu Gly Ile Thr Gly Pro Glu Gly His Val
       210                 215                 220
   Leu Ser His Pro Glu Glu Val Glu Ala Glu Ala Val Tyr Arg Ala Val
   225                 230                 235                 240
   Thr Ile Ala Lys Gln Ala Asn Cys Pro Leu Tyr Val Thr Lys Val Met
                   245                 250                 255
   Ser Lys Gly Ala Ala Asp Ala Ile Ala Gln Ala Lys Arg Arg Gly Val
               260                 265                 270
   Val Val Phe Gly Glu Pro Ile Thr Ala Ser Leu Gly Thr Asp Gly Ser
           275                 280                 285
   His Tyr Trp Ser Lys Asn Trp Ala Lys Ala Ala Ala Phe Val Thr Ser
       290                 295                 300
   Pro Pro Val Asn Pro Asp Pro Thr Thr Ala Asp His Leu Thr Cys Leu
   305                 310                 315                 320
   Leu Ser Ser Gly Asp Leu Gln Val Thr Gly Ser Ala His Cys Thr Phe
                   325                 330                 335
   Thr Thr Ala Gln Lys Ala Val Gly Lys Asp Asn Phe Ala Leu Ile Pro
               340                 345                 350
   Glu Gly Thr Asn Gly Ile Glu Glu Arg Met Ser Met Val Trp Glu Lys
           355                 360                 365
   Cys Val Ala Ser Gly Lys Met Asp Glu Asn Glu Phe Val Ala Val Thr
       370                 375                 380
   Ser Thr Asn Ala Ala Lys Ile Phe Asn Phe Tyr Pro Arg Lys Gly Arg
   385                 390                 395                 400
   Val Ala Val Gly Ser Asp Ala Asp Leu Val Ile Trp Asn Pro Lys Ala
                   405                 410                 415
   Thr Lys Ile Ile Ser Ala Lys Thr His Asn Leu Asn Val Glu Tyr Asn
               420                 425                 430
   Ile Phe Glu Gly Val Glu Cys Arg Gly Ala Pro Ala Val Val Ile Ser
           435                 440                 445
   Gln Gly Arg Val Ala Leu Glu Asp Gly Lys Met Phe Val Thr Pro Gly
       450                 455                 460
   Ala Gly Arg Phe Val Pro Arg Lys Thr Phe Pro Asp Phe Val Tyr Lys
   465                 470                 475                 480
   Arg Ile Lys Ala Arg Asn Arg Leu Ala Glu Ile His Gly Val Pro Arg
                   485                 490                 495
   Gly Leu Tyr Asp Gly Pro Val His Glu Val Met Val Pro Ala Lys Pro
               500                 505                 510
   Gly Ser Gly Ala Pro Ala Arg Ala Ser Cys Pro Gly Lys Ile Ser Val
           515                 520                 525
   Pro Pro Val Arg Asn Leu His Gln Ser Gly Phe Ser Leu Ser Gly Ser
       530                 535                 540
   Gln Ala Asp Asp His Ile Ala Arg Arg Thr Ala Gln Lys Ile Met Ala
   545                 550                 555                 560
   Pro Pro Gly Gly Arg Ser Asn Ile Thr Ser Leu Ser
                   565                 570